## Europäisches Patentamt

## European Patent Office

## Office européen des brevets

(19)

(11) Veröffentlichungsnummer: **0 083 791**
**B1**

(12) **EUROPÄISCHE PATENTSCHRIFT**

(45) Veröffentlichungstag der Patentschrift:
**04.12.85**

(21) Anmeldenummer: **82112017.7**

(22) Anmeldetag: **27.12.82**

(51) Int. Cl.⁴: **B 01 J 21/08, B 01 J 37/02**

(54) Kieselsäurehaltige Formkörper, Verfahren zu ihrer Herstellung und ihre Verwendung.

(30) Priorität: **09.01.82 DE 3200483**

(43) Veröffentlichungstag der Anmeldung:
**20.07.83 Patentblatt 83/29**

(45) Bekanntmachung des Hinweises auf die Patenterteilung:
**04.12.85 Patentblatt 85/49**

(73) Patentinhaber: **BAYER AG, Konzernverwaltung RP Patentabteilung, D-5090 Leverkusen 1 Bayerwerk (DE)**

(72) Erfinder: **Wissner, Adolf, Dr., Am Wasserturm 13, D-5090 Leverkusen 3 (DE)**
Erfinder: **Haydn, Josef, Dr., Leipziger Strasse 27, D-5090 Leverkusen 1 (DE)**
Erfinder: **Birkenstock, Udo, Dr., Schneppersdelle 2, D-4030 Ratingen 8 (DE)**

(84) Benannte Vertragsstaaten:
**BE CH DE FR GB IT LI NL**

(56) Entgegenhaltungen:
**DE - A - 2 647 702**
**FR - A - 2 187 408**
**GB - A - 675 914**
**US - A - 2 643 980**
**US - A - 4 085 131**

BUNDESDRUCKEREI BERLIN

## Beschreibung

Die vorliegende Erfindung betrifft neue kieselsäurehaltige Formkörper, Verfahren zu ihrer Herstellung und ihre Verwendung als Katalysatoren und Trägermaterialien für Katalysatoren.

Kieselsäurehaltige Formkörper werden in großem Ausmaß als Katalysatoren und Trägermaterialien für Katalysatoren verwendet. Eine große Gruppe derartiger Formkörper sind homogene Kieselsäuremassen, z. B. Kugeln mit Durchmessern im Bereich 2 bis 5 mm oder Strangpreßlinge, und werden z. B. durch Eingießen von Kieselsäurehydrosol in nicht mit Wasser mischbare, organische Flüssigkeiten oder durch mechanische Verformung hergestellt (siehe z. B. DE-B-1 197 851 und Firmenmerkblätter: Kali-Chemie Katalysatoren, 1980; Süd-Chemie K-Katalysatoren, 1973; BASF Katalysatoren-Verkaufsprogramm, 1967).

Derartige Formkörper zeigen bei direktem Einsatz als Katalysatoren oder nach dem Belegen mit katalytisch aktiven Stoffen, z. B. durch Aufsprühen oder Tränken, den Nachteil, daß die katalytisch aktiven Zentren mehr oder weniger gleichmäßig im gesamten Formkörper verteilt sind (siehe z. B. US-A-3 743 607). Dadurch können Reaktanden und Reaktionsprodukte im Formkörper festgehalten werden und Anlaß zur Bildung unerwünschter Neben- und Folgereaktionen geben (siehe z. B. Suter, Phthalsäureanhydrid und seine Verwendung, Steinkopf Verlag Darmstadt, S. 20 [1972]). Weiterhin können sich Nebenprodukte und Katalysatorgifte im Inneren der Formkörper anreichern und damit mit üblichen Regenerationsverfahren nur sehr schwer oder überhaupt nicht mehr entfernt werden. Diese negativen Effekte treten besonders stark dann auf, wenn die Formkörper durchgehend porös sind.

Es wurde deshalb schon versucht, Katalysatoren herzustellen, die im Inneren nahezu frei von Poren und/oder katalytisch aktiven Zentren sind. Ein Beispiel hierfür ist der in US-A-1 987 506 beschriebene Katalysator, bei dem 4 bis 6 mm große Körner eines porenarmen oder porenlosen Trägers, wie Korund oder Siliciumcarbid, mit Vanadiumpentoxid überzogen sind. Zu einer Verbesserung dieses Katalysatortyps wird ein Gemisch aus Vanadiumpentoxid und Titandioxid auf inerte, glatte Kugeln aus Porzellan, Steatit oder Quarz aufgebracht (siehe Chemie-Ingenieur-Technik 4, 967 bis 970 [1969]). Bei diesen Katalysatoren ist der katalytisch aktive Bestandteil durch einen niedrigen Schmelzpunkt und die Neigung zur Glasbildung besonders geeignet, um als Überzug aufgebracht zu werden. Es sind auch auf dem gleichen Herstellungsprinzip beruhende Katalysatoren bekannt, bei denen andere katalytisch aktive Bestandteile als Vanadiumpentoxid in reiner Form oder im Gemisch mit Tiandioxid auf einen Träger aufgebracht werden. Alle derartigen Titandioxid enthaltenden Katalysatoren haben den Nachteil, daß sie zusammen mit sonstigen sauren Katalysatorbestandteilen, sauren Reaktanden oder saure Verbindungen enthaltenden Reaktanden eine unzulängliche mechanische Stabilität aufweisen.

Beispielsweise wird in der DE-A-2 909 670 ein Verfahren beschrieben, bei dem katalytisch aktive Substanzen, z. B. Molybdän, Vanadium, Wolfram und/oder Kupfer, als Oxidgemische in Form einer wäßrigen Suspension auf inerte Träger, beispielsweise Siliciumdioxid oder Silikate, gespritzt werden. Dabei muß die Temperatur der Träger unterhalb 100°C liegen, der Träger vor dem Aufsprühvorgang angefeuchtet, ein erhitztes inertes Gas zugeführt und beachtet werden, daß der Wassergehalt des Trägers stets größer ist als derjenige der entstehenden Schale. In allen Fällen entsteht ein Katalysator, dessen äußere Teile nicht porös sind.

In der DE-A-2 716 154 ist die Herstellung eines Katalysators beschrieben, bei dem ein poröser Siliciumdioxid-Träger durch ein spezielles Aufbringungsverfahren so mit Palladium und Gold belegt wird, daß sich die katalytisch aktiven Bestandteile in einer Oberflächenschicht abscheiden, die sich um weniger als 0,5 mm von der Oberfläche erstreckt. Dieses Aufbringungsverfahren ist jedoch sehr aufwendig, nicht allgemein anwendbar und ergibt Katalysatoren, die nach einer Behandlung mit Alkalimetallacetat auch im Inneren aus aktivem Kieselsäurematerial und katalytisch aktiven Substanzen bestehen.

Es wurden nun kieselsäurehaltige Formkörper gefunden, die dadurch gekennzeichnet sind, daß ein Hohlraum oder eine stückige anorganische Grundmasse von einer poröse Kieselsäure enthaltenden Schicht umgeben ist.

Sofern bei den erfindungsgemäßen Formkörpern eine stückige anorganische Grundmasse von einer porösen Kieselsäure enthaltenden Schicht umgeben ist, kann die anorganische Grundmasse aus den verschiedensten Materialien bestehen, die nicht, wenig oder sehr porös sein können und ausreichend fest und inert sind. Als Beispiele seien genannt: Siliciumdioxid und Silikate, wie Natriumaluminiumsilikate, Magnesiumsilikate (z. B. Steatite), Zirkoniumsilikate, Silikatgläser, Quarz, Calciumzirkoniumsilikate und Aluminiumsilikate; Aluminiumoxid und Aluminiumoxid enthaltende Produkte, wie Korund, $\alpha$-Aluminiumoxid, $\gamma$-Aluminiumoxid, Calciumaluminat und Spinelle, wie Lithium-Spinelle; sonstige oxidische Materialien, wie Titandioxid, Zirkondioxid, Thoriumdioxid, Magnesiumoxid und Zinkoxid; metallische Materialien; wie Stähle, Eisen und Kupfer; Gesteine, wie Granit, Basalt, natürliche Eisenoxide und Bimsstein; Materialien, die üblicherweise als Füllkörper für Kolonnen Anwendung finden, wie Raschigringe, Berlsättel und Scherben aus beispielsweise Glas, Porzellan oder Ton; sonstige anorganische Materialien wie Carbide, z. B. Siliciumcarbid, Carbonate, z. B. Calciumcarbonat, sowie Schlacken, Aschen, Kohlen und Blähton.

Vorzugsweise besteht die anorganische Grundmasse aus Materialien mit niedriger Saugfähigkeit und niedriger BET-Oberfläche. Vorzugsweise beträgt die Saugfähigkeit weniger als 10, besonders bevorzugt weniger als 5 g Wasser pro 100 g, und die BET-Oberfläche liegt vorzugsweise unter 5 m²/g. Wenn anorganische Grundmassen mit höherer Saugfähigkeit und/oder höherer BET-Oberfläche vorliegen, so ist es vorteilhaft, zwischen der anorganischen Grundmasse und der poröse Kieselsäure enthaltenden Schicht eine weitere Schicht anzuordnen, welche die Porosität der anorganischen Grundmasse an deren Oberfläche so weit herabsetzt, daß dort die bevorzugten Bereiche für die Saugfähigkeit und die BET-Oberfläche vorliegen. Einzelheiten hierzu werden später beschrieben. Weiterhin besteht die anorganische Grundmasse vorzugsweise aus Silikaten, insbesondere aus Magnesiumsilikaten in Form von Steatiten, Siliciumdioxid, Aluminiumoxiden, Stählen, Eisen, erschmolzenen Aschen, erschmolzenen Schlacken oder Füllkörpern.

Die Kristallstruktur der anorganischen Grundmasse hat keine besondere Bedeutung. Es kann sich um kristalline oder amorphe anorganische Grundmassen handeln.

Die Form und Teilchengröße der stückigen anorganischen Grundmasse ist ebenfalls nicht von besonderer Bedeutung. Beispielsweise kann es sich um tablettierte, körnige oder sonstige stückige anorganische Grundmassen handeln, sowie um Extrudate, Kugeln, Bundkugeln, Granulate, Röhrchen, Stäbchen, Zylinder, Mahlgut und andere beliebig geformte Partikel, beispielsweise um Ringe. Insbesondere metallische Materialien können auch in Form von Drähten, Drahtnetzen, Schrauben, Muttern und sonstigen geformten Kleinteilen vorliegen. Vorzugsweise haben die Partikel der anorganischen Grundmassen Abmessungen im Bereich von 0,5 bis 15 mm. Besonders bevorzugt sind Kugeln, Granulate und Mahlgut mit einem mittleren Durchmesser im Bereich von 0,5 bis 15 mm, ganz besonders bevorzugt sind Kugeln, Granulate und Mahlgut mit einem mittleren Durchmesser von 2 bis 10 mm. Die anorganische Grundmasse kann auch aus Partikeln verschiedener Art, z. B. Steatit und Schlacke, und verschiedener Form, z. B. Kugeln und Mahlgut, bestehen.

Bei den erfindungsgemäßen Formkörpern ist entweder eine der zuvor beschriebenen anorganischen Grundmassen oder ein Hohlraum von einer poröse Kieselsäure enthaltenden Schicht umgeben. Die Dicke dieser Schicht kann in weiten Grenzen variieren, beispielsweise von 10 bis 3000 µm. Vorzugsweise beträgt die mittlere Dicke dieser Schicht 20 bis 2000 µm. Die Schichtdicke kann von Partikel zu Partikel und auch innerhalb eines Partikels variieren. Letzteres insbesondere dann, wenn unregelmäßig geformte anorganische Grundmassen, wie z. B. Asche oder Mahlgut, vorliegen.

In einer besonderen Ausführungsform der Erfindung enthält die poröse Kieselsäure enthaltende Schicht zusätzlich katalytisch aktive Substanzen oder Vorläufer davon. Derartige katalytisch aktive Substanzen oder Vorläufer davon können von der verschiedensten Art sein. Es kann sich hierbei beispielsweise um Metalle, Metallverbindungen, Nichtmetalle, Nichtmetallverbindungen, Komplexverbindungen, Ionenaustauscher, Kohlen und/oder Zeolithe handeln. Beispielsweise kommen hierfür Elemente und/oder Verbindungen von Elementen der 1. bis 6. Hauptgruppe und/oder der 1. bis 8. Nebengruppe des Periodensystems sowie der Seltenen Erden und/oder Aktiniden in Frage. Im einzelnen seien beispielsweise folgende Elemente genannt: Lithium, Natrium, Kalium, Rubidium, Cäsium, Beryllium, Magnesium, Calcium, Strontium, Barium, Titan, Zirkonium, Hafnium, Vanadium, Niob, Tantal, Chrom, Molybdän, Wolfram, Mangan, Rhenium, Eisen, Ruthenium, Osmium, Kobalt, Iridium, Rhodium, Nickel, Palladium, Platin, Kupfer, Silber, Gold, Zink, Cadmium, Quecksilber, Bor, Aluminium, Kohlenstoff, Silicium, Zinn, Blei, Phosphor, Arsen, Antimon, Bismut, Cer, Scandium, Yttrium, Gallium, Thallium, Germanium, Samarium, Thorium und/oder Uran. Diese Elemente können als solche oder in Form von Verbindungen vorliegen, wobei selbstverständlich solche Elemente und Verbindungen ausgeschlossen sind, die nicht ausreichend stabil sind. Die poröse, Kieselsäure enthaltende Schicht kann auch Metalle des Raney-Typs, z. B. Raney-Nickel, Raney-Eisen und/oder Raney-Kobalt, enthalten oder die Vorläufer davon, z. B. fein verteilte Raney-Legierungen.

Als Verbindungen und Komplexverbindungen kommen beispielsweise Salze und/oder Komplexverbindungen der vorgenannten Elemente in Frage, vorzugsweise Sauerstoff enthaltende Verbindungen, wie Oxide, Hydroxide, Sulfate, Carbonate, Carboxylate (z. B. Acetate), Nitrate, Phosphate, Silikate, Borate und Aluminate, aber auch Cyanide, Fluoride, Chloride, Bromide, Phthalocyanide, Acetylacetonate und sonstige Komplexverbindungen. Geeignete Verbindungen sind beispielsweise in A. F. Wells, Structural Inorganic Chemistry, 3. Auflage, Oxford at the Clarendon Press (1967), und in D. Brown, J. H. Canterford und R. Coltan, Halides of the Transition Elements, Band 1 bis 3, John Wiley & Sons, London (1968), aufgeführt.

Vorzugsweise kommen folgende Verbindungen in Frage: Lithiumsulfat, Natriumsulfat, Natriumacetat, Kaliumsulfat, Kaliumacetat, Rubidiumsulfat, Cäsiumsulfat, Lithiumcarbonat, Natriumcarbonat, Kaliumcarbonat, Rubidiumcarbonat, Cäsiumcarbonat sowie die entsprechenden Hydrogencarbonate, Lithiumoxid, Natriumoxid, Kaliumoxid, Cäsiumoxid, Rubidiumoxid, Berylliumoxid, Magnesiumoxid, Calciumoxid, Strontiumoxid, Magnesiumcarbonat, Calciumcarbonat, Strontiumcarbonat sowie die entsprechenden Hydrogencarbonate, Vanadiumpentoxid, Vanadylsulfat, Vanadyloxalat, Chromtrioxid, Molybdäntrioxid, Wolframtrioxid, Manganoxid, Mangandioxid, Rheniumoxid, Eisen-(II)-oxid, Eisen-(III)-oxid, Eisen-(II/III)-oxid,Rutheniumdioxid, Kobalt-(II)-oxid, Kobalt-(II/III)-oxid, Nickeloxid, Kupferoxid, Zinkoxid, Boroxid, Aluminiumoxid, Zinndioxid, Bleimonoxid, Bleidioxid, Blei-(II/IV)-oxid, Phosphorpentoxid, Cerdioxid, Antimontrioxid, Antimonpentoxid, Arsensäure, Arsentrioxid, Berylliumnitrat, Bleinitrat, Bleiacetat, Cadmiumnitrat, Cadmiumsulfat, Cer-(III)-nitrat, Cer-(IV)-sulfat, Chrom-(III)-nitrat, Eisen-(III)-nitrat, Eisen-(II)-sulfat, Eisen-(III)-sulfat, Kaliumdichromat, Kaliumchromat, Kali-

umhexacyanoferrat-(II), Kaliumhexacyanoferrat-(III), Kaliumtetrahydroxyantimonat, Dikaliumhydrogenphosphat, Kobaltnitrat, Kupfernitrat, Kupfersulfat, Lanthannitrat, Mangan-(II)-nitrat, Natriumarsenat, Natriummolybdat, Natriumvanadat, Natriumwolframat, Nickelnitrat, Nickelsulfat, Quecksilber-(II)-nitrat, Silbernitrat, Thalliumnitrat, Thorium-(IV)-nitrat, Uranylnitrat, Bismutnitrat, Zinknitrat, Zirkonylchlorid, Hexachloroplatinsäure, Kaliumhexachloroplatinat, Natriumtetrachloropalladat, Palladiumchlorid, Palladiumbromid, Palladiumjodid, Kaliumhexachloropalladinat, Palladiumacetat, Palladiumnitrat, Palladiumsulfat, Palladiumdiamindichlorid, Rhodiumtrichlorid, Rhodiumtrinitrat, Hexachloroiridiumsäure, Iridiumtrichlorid, Rutheniumtrichlorid, Bismuttrichlorid, Chromtrichlorid, Samariumtrichlorid und/oder Nioboxychlorid sowie Phosphorsäure, Hydrogenphosphate, Dihydrogenphosphate, Pyrophosphate, z. B. Vanadylphosphate, Vanadylpyrophosphate, Eisenphosphate, Eisenpyrophosphate und Kupferphosphate.

Von besonderem Interesse sind Kohlen, Zeolithe, Ionenaustauscher, Aluminiumoxide und Kieselsäuren.

Bei den Kohlen handelt es sich vorzugsweise um pulverförmige, kohlenstoffhaltige Systeme, z. B. Aktivkohlen, wie sie in »Lurgi Schnellinformation (T 1091/8.75), Pulverförmige Aktivkohlen« beschrieben sind. Geeignete Aktivkohlen haben z. B. die Zusammensetzung: Kohlenstoff 83 bis 93 Gew.-%, Wasserstoff 1 bis 3 Gew.-%, Sauerstoff 0,2 bis 10 Gew.-%, Stickstoffspuren, Schwefelspuren und enthalten ergänzend zu 100 Gew.-% Asche und weisen Oberflächen von bis zu 1500 m$^2$/g und größer auf. Sie weisen z. B. eine Korngröße von unter 1 mm, vorzugsweise eine Mahlfeinheit von ca. 20 Gew.-% über 75 µm auf.

Bei den Zeolithen kann es sich um natürlich vorkommende oder synthetisch hergestellte handeln, z. B. um Systeme, die aus Kieselsäure und Tonerde in Verbindung mit Alkali- oder Erdalkalioxiden bestehen. Geeignete Zeolithe sind ausführlich in R. M. Barrer, Molecular Sieves, Society of Chemical Industry, London, S. 39 (1968), und in der Merkschrift der Bayer AG »Bayer-Zeolith«, Ausgabe 1. Jan. 1978, beschrieben. Geeignete Zeolithe haben z. B. folgende Merkmale: Porenweite 3 bis 9 Å, Körnung 1 bis 6 mm und Schüttgewicht 600 bis 750 g/l und sind bevorzugt feine Pulver mit einer Korngröße von wesentlich weniger als 1 mm.

Bei den Ionenaustauschern kann es sich um Kunstharze handeln, die aufgrund ihres chemischen Aufbaus und einer porösen, wasserdurchlässigen Struktur äußerst reaktionsfähig im Sinne einer Bindung von Fremdionen über Kationen- oder Anionenaustausch wirksam sind. Geeignete Ionenaustauscher sind z. B. beschrieben in der Merkschrift der Bayer AG »Lewatit®/Lewasorb®«, Produktinformationen, Ausgabe Januar 1981. Sowohl Kationen- als auch Anionenaustauscher, beispielsweise die Typen starksauer-gelförmig Na- oder H-Form, starksauer-makroporös Na-Form, schwachsauer-makroporös H-Form, starkbasisch-gelförmig Cl- oder OH-Form, starkbasisch-makroporös Cl-Form, schwachbasisch-makroporös Cl-Form und schwachbasisch-makroporös OH- oder OH-/Cl-Form sind geeignet. Die Korngröße der Ionenaustauscher soll möglichst weniger als 1 mm, vorzugsweise weniger als 0,5 mm betragen.

Bei den Aluminiumoxiden kann es sich um alle Zwischenstufen handeln, von den Aluminiumoxid-Hydraten bis zu reinem Aluminiumoxid, wie sie z. B. in den Firmenschriften von Rhône-Poulenc, »Activated Alumina« und »Alumina Catalyst Carriers Spheralite®«, beschrieben sind. Beispielsweise kommen Aluminiumoxide mit über 95 Gew.-% Al$_2$O$_3$-Gehalt in Frage. Es kann sich aber auch um Aluminiumoxide mit geringeren Al$_2$O$_3$-Gehalten handeln. Vorzugsweise kommt Aluminiumoxid als pulverförmiges Material mit einer Korngröße unter 1 mm, vorzugsweise unter 0,5 mm, in Frage.

Bei den Kieselsäuren kann es sich um die weiter unten beschriebenen feinporigen, wasserunlöslichen Kieselsäuren handeln.

Die vorliegende Erfindung betrifft weiterhin ein Verfahren zur Herstellung kieselsäurehaltiger Formkörper der vorbeschriebenen Art, das dadurch gekennzeichnet ist, daß man Kieselsol oder ein Gemisch, enthaltend Wasser, Kieselsol und/oder Wasserglas und gegebenenfalls feinpulvrige, wasserunlösliche Kieselsäure und/oder Porosierungsmittel, auf eine stückige Grundmasse aufbringt, wobei die Temperatur der Grundmasse zu Beginn des Aufbringens des Kieselsols oder des Gemisches unter dem Erweichungspunkt der Grundmasse und im weiteren Verlauf des Aufbringens des Kieselsols oder des Gemisches über der Siedetemperatur des Wassers liegt, das Kieselsol oder das Gemisch so zugegeben wird, daß das Wasser schnell verdampft und der Wassergehalt der Grundmasse stets unter 5 Gew.-% liegt und, im Falle des Einsatzes von organischen Grundmassen, diese anschließend durch Erhitzen auf Temperaturen im Bereich von 200 bis 1300° C entfernt.

Als Grundmasse für die Herstellung der erfindungsgemäßen Formkörper sind anorganische und organische Materialien geeignet. Als anorganische Grundmassen sind die zuvor beschriebenen geeignet, wobei zu beachten ist, daß deren durchschnittliche Restfeuchte zu Beginn und während des Aufbringens des Kieselsols oder des Gemisches möglichst niedrig gehalten wird, d. h. stets unter 5 Gew.-%, vorzugsweise wesentlich niedriger.

Als organische Grundmassen sind solche geeignet, die nicht, wenig oder sehr porös, ausreichend fest und in Wasser schwer löslich sind. Bevorzugt sind feste polymere Stoffe, beispielsweise Polystyrole, Polycarbonate, Polyolefine, Cellulose, Cellulosederivate, Polyurethane, Polyacrylnitrile, Acrylnitril-Styrol-Butadien-Mischpolymerisate, Polyester, Polyvinylchloride, Polyfluorethylene, Polyfluorethylenderivate, Polyamide, Epoxidharze und Polykondensate, beispielsweise solche aus Phenol und

4

Formaldehyd. Vorzugsweise besteht die organische Grundmasse aus nicht porösen Materialien, die einen Erweichungspunkt über 60°C aufweisen. Die Form und Größe organischer Grundmassen kann derjenigen der zuvor beschriebenen anorganischen Grundmassen entsprechen. Die in Betracht kommenden organischen Grundmassen sind im allgemeinen wasserabstoßend, so daß deren Wassergehalt vor und während der Aufbringung des Gemisches im allgemeinen nicht besonders beachtet werden muß.

Die organischen Grundmassen werden nach dem Aufbringen des Kieselsols oder des Gemisches durch Erhitzen entfernt. Dadurch entstehen Hohlräume, die von einer poröse Kieselsäure enthaltenden Schicht umgeben sind.

Als Grundmasse kann auch ein Gemisch verschiedener organischer Grundmassen, z. B. Polystyrol und Polycarbonat, und auch ein Gemisch anorganischer und organischer Grundmassen, z. B. Steatit und Polycarbonat, eingesetzt werden. Es ist auch möglich, organische Grundmassen einzusetzen, die anorganische Bestandteile enthalten, z. B. mit Glasfasern verstärkte Polymere.

Auf die Grundmasse kann beispielsweise folgendes aufgebracht werden:

— nur Kieselsol oder
— Gemische, die neben Wasser folgende Bestandteile enthalten:
   a)   Kieselsol,
   b)   Wasserglas,
   c)   Kieselsol und Wasserglas in beliebigen Mischungsverhältnissen,
   d)   10 bis 90 Gew.-% Kieselsol und ergänzend auf 100 Gew.-% feinpulvrige, wasserunlösliche Kieselsäure, jeweils berechnet und bezogen auf wasserfreies $SiO_2$,
   e)   10 bis 60 Gew.-% Wasserglas und ergänzend auf 100 Gew.-% feinpulvrige, wasserunlösliche Kieselsäure, jeweils berechnet und bezogen auf wasserfreies $SiO_2$,
   f)   10 bis 90 Gew.-% Kieselsol und Wasserglas, wobei der Wasserglasanteil maximal 60 Gew.-% der Mischung beträgt, und ergänzend zu 100 Gew.-% feinpulvrige, wasserunlösliche Kieselsäure, jeweils berechnet und bezogen auf wasserfreies $SiO_2$.

Gemische, die als silikatische Komponente nur Wasserglas enthalten, werden im allgemeinen nur dann verwendet, wenn man, beispielsweise beim Einsatz von stark porösen Grundmassen oder solchen mit sehr glatten Oberflächen, eine erste Schicht zur Herabsetzung der Porosität und/oder Verbesserung der Haftfestigkeit erzeugen will und danach eine oder mehrere Schichten aufbringt, die weitere oder andere silikatische Komponenten enthalten. Bei der alleinigen Verwendung von Kieselsol und bei allen anderen Wasser enthaltenden Gemischen ist es ohne besondere Bedeutung, auf welche Art von Grundmasse sie aufgetragen werden, ob sie zur Erzeugung von Einfach- oder Mehrfachbeschichtungen verwendet werden und ob einer erzeugten Schicht noch eine oder mehrere weitere folgen. Bevorzugt werden Kieselsol und Gemische eingesetzt, wie sie unter d), e) und f) beschrieben sind, gegebenenfalls zusammen mit Porosierungsmitteln.

Sofern bei der Herstellung der aufzubringenden Komponenten oder Komponentenmischungen Wasser verwendet wird, können dem Wasser als Hilfsmedium gegebenenfalls die verschiedensten organischen Lösungsmittel zugesetzt werden, wobei es nicht erforderlich ist, immer eine homogene flüssige Phase einzustellen. Es kommen z. B. folgen organische Lösungsmittel in Betracht: aliphatische, cycloaliphatische oder aromatische sowie heterocyclische Verbindungen, die auch substituiert sein können. Als aliphatische Kohlenwasserstoffe sind geradkettige oder verzweigte Kohlenwasserstoffe mit 1—12, bevorzugt mit 5—8 Kohlenwasserstoffatomen geeignet. Als cyclische Kohlenwasserstoffe kommen solche mit 5—7, vorzugsweise mit 6 Kohlenstoffatomen im Ringsystem in Betracht. Als heterocyclische Verbindungen kommen solche mit 5—7, vorzugsweise 6 Atomen im Ringsystem in Betracht. Als heterocyclische Verbindungen sind bevorzugt 5- und 6gliedrige Systeme, die als Heteroatom Sauerstoff und/oder Stickstoff enthalten können, geeignet.

Die zugesetzten Lösungsmittel können Substituenten aufweisen wie Halogenatome, z. B. Fluor, Chlor, Brom; Hydroxyl-, Amino-, Sulfonsäure-, Carboxyl-Gruppen sowie deren Ester; $C_1$—$C_4$-Alkoxy-Gruppen und $C_1$—$C_{12}$-Alkylreste. Besonders bevorzugt als organische Lösungsmittel kommen in Betracht Kohlenwasserstoffe wie Hexan, Cyclohexan, Benzol, Toluol, Xylol; Alkohole wie Methanol, Ethanol, Propanol, Isopropanol, Butanol, Amylalkohol, Ethylenglykol, Glycerin, Cyclohexanol; Ether wie Ethylenglykolmono- und Ethylenglykoldiethylether, Ethylenglykolmonotolylether, Triethylglykolmethylether; Ketone wie Aceton; Amine wie Ethylamin, Cyclohexylamin, Ethylendiamin; Phenole wie Phenol, 3-Acetoxyphenol, Resorcin, Brenzcatechin, sowie Gemische und Mischungen dieser Verbindungen in den verschiedensten Zusammensetzungen.

Sofern organische Grundmassen und organische Lösungsmittel eingesetzt werden, ist zu beachten, daß nur solche organische Lösungsmittel verwendet werden, die die jeweilige organische Grundmasse nicht oder nur geringfügig zu lösen vermögen.

In besonderen Fällen können auch Mischungen, bei denen das flüssige Medium nur ganz geringe Anteile Wasser enthält, eingesetzt werden.

Alle auf die Grundmasse aufzubringenden silikathaltigen Stoffe oder Gemische können wahlweise zusätzlich ein oder mehrere Porosierungsmittel enthalten, beispielsweise 0 bis 100, vorzugsweise 0 bis 50 Gew.-%, bezogen auf alle vorliegenden, als wasserfreies $SiO_2$ berechneten Kieselsäurebestandteile.

Als Kieselsol können wäßrige, kolloidale Kieselsäurelösungen in den verschiedensten Beschaffenheiten eingesetzt werden, wie sie beispielsweise in dem Merkblatt Bayer-Anorganika, Silica-Sol, Order Nr. AC 10006 e vom 1. Feb. 1973, beschrieben sind. Vorzugsweise enthält das Kieselsol 15 bis 45 Gew.-% $SiO_2$ und unter 0,5 Gew.-% $Na_2O$ und weist einen pH-Wert im Bereich von 3,4 bis 10, eine Dichte im Bereich von 1,09 bis 1,33 g/cm$^3$ und eine Teilchengröße im Bereich von 7 bis 30 μm auf. Geeignete Kieselsole sind im Handel erhältlich.

Als Wasserglas kommen wäßrige Lösungen von Alkalisilikaten in Frage, insbesondere handelsübliche Alkalisilikat-Lösungen, die unter den Bezeichnungen Kaliwasserglas und Natronwasserglas erhältlich sind und $SiO_2$-Gehalte bis zu 30 Gew.-% aufweisen. Geeignet sind auch Wasserglasarten, wie sie in Hollemann-Wiberg, Lehrbuch der anorganischen Chemie, 71. bis 80. Auflage, Verlag Walter de Gruyter, Berlin, S. 497 (1971), beschrieben sind. Als Wasserglas können jedoch auch feste Alkali-metasilikate, z. B. Kaliummetasilikat, eingesetzt werden, die $SiO_2$-Gehalte bis zu 75 Gew.-% aufweisen. Es kann Kieselsol allein eingesetzt werden, aber auch Mischung aus Wasser und Kieselsol, Mischungen aus Wasser und Wasserglas und Mischungen aus Wasser, Kieselsol und Wasserglas.

Als feinpulvrige, wasserunlösliche Kieselsäure kommen beispielsweise die verschiedensten amorphen oder kristallinen Kieselsäuren in Frage, wobei die Primär-Teilchengröße beispielsweise 1 bis 200 nm und die Agglomeratgröße beispielsweise 0,5 bis 100 μm sind.

Solche Kieselsäuren sind handelsüblich und beispielsweise in H. Ferch, Chemie-Ingenieur-Technik, 48, S. 922 ff. (1976), beschrieben. Im allgemeinen handelt es sich dabei um synthetische Kieselsäuren, die auf verschiedene Weise hergestellt sein können, beispielsweise nach der Flammhydrolyse-Methode aus Siliciumtetrachlorid, Wasserstoff und Sauerstoff oder nach dem Lichtbogenverfahren aus Quarz und Koks. Von besonderer Bedeutung sind die nach dem sogenannten »Naß-Verfahren« hergestellten Kieselsäuren, bei denen aus Wasserglas und Säure durch Fällung und anschließende Trocknung oder nach dem Hydrothermalverfahren aus Sand und Kalk Kieselsäure erhalten wird. Alle nach diesem Verfahren erhältlichen Kieselsäureprodukte sind im Handel erhältlich.

Vorzugsweise weist die feinpulvrige, wasserunlösliche Kieselsäure folgende Charakteristika auf: Spezifische Oberfläche 30 bis 2000 m$^2$/g, Primärteilchengröße 3 bis 1000 nm, Agglomeratgröße 1 bis 40 μm, Dichte ca. 2,0 g/cm$^3$, Stampfvolumen (nach DIN 53 194) 100 bis 2000 ml/100 g, Trocknungsverlust (nach DIN 53 198) 3 bis 7%, Glühverlust (nach DIN 55 921) 3 bis 15%, pH-Wert (nach DIN 53 200) 2 bis 9, vorherrschender Porendurchmesser über 200 Å und Gehalt an $SiO_2$ (bezogen auf Trockensubstanz) über 93%.

Die einzusetzenden feinpulvrigen, wasserunlöslichen Kieselsäuren können geringe Mengen an Verunreinigungen enthalten, beispielsweise bis zu 1 Gew.-% Aluminium (berechnet als $Al_2O_3$), bis zu 0,5 Gew.-% Eisen (berechnet als $Fe_2O_3$) und bis zu 2 Gew.-% Schwefel (berechnet als $SO_3$), jeweils bezogen auf das Trockengewicht der Kieselsäure.

Vorzugsweise sind weniger als 0,5 Gew.-% Aluminium, weniger als 0,1 Gew.-% Eisen und weniger als 1 Gew.-% Schwefel enthalten, jeweils berechnet und bezogen wie vorstehend angegeben.

Als feinpulvrige, wasserunlösliche Kieselsäure kann auch Kieselgur eingesetzt werden.

Als Kieselgur kommt das unter dieser Bezeichnung handelsübliche Naturprodukt in Frage, wie es beispielsweise in Römpps Chemielexikon, Band H bis L, 7. Auflage, Franckhsche Verlagshandlung W. Keller & Co., Stuttgart, S. 1770 (1973), beschrieben ist. Vorzugsweise handelt es sich dabei um ein feinkörniges, leichtes Pulver, das 70 bis 90 Gew.-% amorphe Kieselsäure, 3 bis 12 Gew.-% Wasser und geringe Beimengungen von Oxiden anderer Elemente, z. B. von Aluminium und Eisen, enthält.

Das Kieselsol und die Wasser, Kieselsol und/oder Wasserglas enthaltenden Gemische, die jeweils gegebenenfalls Zusätze von Lösungsmitteln und/oder feinpulvriger, wasserunlöslicher Kieselsäure enthalten, bedürfen nicht zwingend des Zusatzes von Porosierungsmitteln. Insbesondere, wenn größere Anteile Kieselgur vorliegen, kann auf Porosierungsmittel verzichtet werden, da Kieselgur eine auflockernde Wirkung auf die Kieselsäure enthaltende Schicht ausübt. Vorzugsweise wird jedoch unabhängig von der Gegenwart von Kieselgur eines oder mehrere Porosierungsmittel zugesetzt.

Als Porosierungsmittel snd die zuvor beschriebenen Aktivkohlen, Ruße und Graphit geeignet sowie Stoffe, die sich thermisch leicht und weitgehend rückstandsfrei zersetzen lassen. Solche Porosierungsmittel sind an sich bekannt. Es kommen beispielsweise organische und/oder anorganische Stoffe in Frage, wie Ammoniumsalze, aliphatische Alkohole der Summenformel $C_nH_{2n+2}O$, aliphatische Di-, Tri- und Polyhydroxyverbindungen, aliphatische Carbonsäuren der Summenformel $C_nH_{2n}O_2$ (auch in Form ihrer Salze, Ester und sonstiger Derivate), aliphatische Dicarbonsäuren der Summenformel $C_nH_{2n-2}O_4$, höhere aliphatische Carbonsäuren und Zucker (auch in Form von Derivaten und Polysacchariden) sowie leicht und vollständig zersetzbare Polymere, wie Polystyrol.

Als Porosierungsmittel geeignete Einzelverbindungen bzw. -stoffe sind beispielsweise Ammoniumcarbonat, -hydrogencarbonat, -nitrat, -acetat, -formiat, -oxalat, Ethylenglykol, Glycerin, Zuckeralkohole, Essigsäure, Propionsäure, Oxalsäure, Natriumacetat, Malonsäure, Ascorbinsäure, Weinsäure, Zitronensäure, Glukose, Saccharose, Stärke, Zellulose und Graphit.

Vorzugsweise kommen als Porosierungsmittel Ammoniumsalze, Oxalsäure und Graphit zum Einsatz.

Bei dem Graphit kann es sich um natürlichen oder synthetischen Graphit handeln. Beispielsweise kann der Graphit einen Kohlenstoffgehalt von 70 bis 90 Gew.-%, einen Aschegehalt von 10 bis 30 Gew.-% und eine Teilchengröße aufweisen, die im wesentlichen unter 0,1 mm liegt. Die Ascheanteile enthalten im allgemeinen im wesentlichen Kieselsäure, daneben Alkali- und Erdalkalioxide sowie gegebenenfalls Oxide von Eisen und/oder sonstigen Übergangsmetallen.

In der auf die Grundmasse aufzubringenden Masse können die Anteile von Kieselsol, Wasser und/oder Wasserglas und gegebenenfalls feinpulvriger wasserunlöslicher Kieselsäure und/oder Porosierungsmitteln in weiten Grenzen schwanken. Wenn man alle vorliegenden Kieselsäurebestandteile als wasserfreies $SiO_2$ berechnet, so kann, hierauf bezogen, der Anteil an Kieselsol z. B. 10 bis 100 Gew.-% betragen, wenn kein Wasserglas eingesetzt wird. Wird Kieselsol und Wasserglas eingesetzt, so kann deren Anteil, alle vorliegenden Kieselsäurebestandteile als wasserfreies $SiO_2$ berechnet, gemeinsam z. B. 20 bis 80 Gew.-% betragen, wobei der Wasserglasanteil daran vorzugsweise 55 Gew.-% nicht übersteigt. Die auf die Grundmasse aufzubringende Masse soll so viel Wasser, gegebenenfalls mit Lösungsmittelzusätzen, enthalten, daß sie dünnflüssig ist. Beispielsweise kann sie, bezogen auf alle vorliegenden, als wasserfreies $SiO_2$ berechneten Kieselsäurebestandteile, 30 bis 95 Gew.-%, vorzugsweise 40 bis 85 Gew.-%, Wasser enthalten.

Durch Variation der Zusammensetzung der auf die Grundmasse aufzubringenden Masse im Rahmen der angegebenen Grenzen lassen sich die physikalischen Parameter der sich bildenden kieselsäurehaltigen Schicht beeinflussen, z. B. das Porenvolumen, der (mittlere) Porendurchmesser, die Porenradienverteilung, die Porosität, die scheinbare Dichte, die wahre Dichte, der Grobporenanteil, der Feinporenanteil, die Saugfähigkeit, die Abriebfestigkeit, die mechanische Festigkeit, die spezifische (BET-)Oberfläche, die spezifische aktive Oberfläche und die Oberflächenacidität.

Das Aufbringen dieser Masse auf die Grundmasse kann nach an sich bekannten Methoden erfolgen, beispielsweise mittels Gieß-, Sprüh- oder Granulierverfahren, wobei sich die Partikel der Grundmasse vorzugsweise in ständiger Eigenbewegung befinden und wobei eine gleichmäßige und konstante Durchmischung angestrebt wird. Wesentlich hierbei ist, daß einerseits die Temperatur der Grundmasse zu Beginn des Aufbringens des Kieselsols oder des Gemisches unter dem Erweichungspunkt der Grundmasse liegt und andererseits, daß das mit dem Kieselsol oder dem Gemisch darauf aufgebrachte Wasser schnell verdampft.

Beim Einsatz von anorganischen Grundmassen, die im allgemeinen hohe Erweichungs- bzw. Schmelzpunkte aufweisen, hält man deshalb deren Temperatur während des gesamten Aufbringens von Kieselsol oder der zuvor beschriebenen Gemische vorzugsweise oberhalb der Siedetemperatur des Wassers, d. h. beim Arbeiten bei Normaldruck oberhalb 100°C. Bevorzugt sind hier Temperaturen im Bereich von 105 bis 800°C, besonders bevorzugt solche im Bereich von 110 bis 400°C, ganz besonders bevorzugt solche im Bereich von 110 bis 300°C.

Organische Grundmassen können Erweichungspunkte unterhalb der Siedetemperatur des Wassers aufweisen. Im Falle des Einsatzes von organischen Grundmassen hält man deshalb deren Temperatur zu Beginn des Aufbringens von Kieselsol oder der zuvor beschriebenen Gemische unter dem Erweichungspunkt, auch wenn dabei die Siedetemperatur des Wassers unterschritten wird.

Das Aufbringen von Kieselsol oder der zuvor beschriebenen Gemische muß dann gegebenenfalls langsamer erfolgen, damit eine schnelle Verdampfung des Wassers gewährleistet bleibt. Nachdem die organische Grundmasse gerade eben mit einer kieselsäurehaltigen Schicht überzogen ist, erhöht man ihre Temperatur auf Werte oberhalb der Siedetemperatur des Wassers und führt das Aufbringen von Kieselsol oder der zuvor beschriebenen Gemische bei Temperaturen oberhalb der Siedetemperatur des Wassers zu Ende. Die dann anwendbare maximale Temperatur hängt von der Art der organischen Grundmasse ab. Im allgemeinen arbeitet man hier bei Temperaturen im Bereich von 105 bis 300°C, vorzugsweise 110 bis 220°C. Auf organische Grundmassen, die einen Erweichungspunkt von oberhalb ca. 110°C aufweisen, bringt man Kieselsol oder das Gemisch vorzugsweise durchgehend bei Temperaturen oberhalb der Siedetemperatur des Wassers auf.

Wenn die aufzubringende Masse (Kieselsol oder eines der zuvor beschriebenen Gemische) neben Wasser wesentliche Anteile höher als Wasser siedender Lösungsmittel enthält, so wählt man die Temperatur der Grundmasse vorzugsweise höher als den Siedepunkt des am höchsten siedenden Lösungsmittels. Weiterhin ist zu beachten, daß die Partikel der Grundmasse vor Beginn des Auftragens eine Restfeuchte von unter 5 Gew.-%, vorzugsweise unter 1 Gew.-% aufweisen.

Wenn man für das Aufbringen der Masse auf die Grundmasse ein Sprühverfahren anwendet, so kann man beispielsweise die Grundmasse in einer heizbaren Dragiertrommel vorlegen und mittels Düsen, z. B. Kegeldüsen, Hohldüsen, Schlickdüsen, Einstoffdüsen oder Mehrstoffdüsen, die aufzubringende Masse aufbringen. Wenn man ein Gießverfahren anwendet, so kann man die aufzubringende Masse beispielsweise auf die bewegte Grundmasse auftropfen. Bei Granulierverfahren werden beispielsweise die Grundmasse und eine fein verteilte, feste, katalytisch aktive Substanz oder ein Vorläufer davon mit Hilfe von Kieselsol oder eines der zuvor beschriebenen Gemische granuliert. Vorzugsweise wendet man Sprühverfahren an.

Die Temperatur der aufzubringenden Masse ist im allgemeinen nicht kritisch. Sie sollte jedoch nicht so hoch liegen, daß schon vor dem Aufbringen auf die Grundmasse wesentliche Mengen Wasser verdampfen. Geeignete Temperaturen sind deshalb beispielsweise solche im Bereich von 10 bis 50°C.

Es ist wesentlich, die aufzubringende Masse so auf die Grundmasse aufzubringen, daß das in ihr

7

enthaltene Wasser schnell verdampft. Der Wassergehalt der Grundmasse und gegebenenfalls bereits darauf aufgebrachter Kieselsäure hat stets unter 5 Gew.-% zu betragen. Vorzugsweise sollte er stets unter 1 Gew.-% betragen. Auf diese Weise erreicht man eine gute Haftung der aufgebrachten Schicht, insbesondere beim Einsatz anorganischer Grundmassen.

Beispielsweise kann man beim Aufbringen der Masse auf eine anorganische Grundmasse so verfahren, daß man die Grundmasse in einer heizbaren Dragiertrommel auf etwa 200 bis 250°C aufheizt, dann mit der kieselsäurehaltigen Masse besprüht, bis die Temperatur der Schüttung auf etwa 105 bis 150°C gefallen ist, dann die Schüttung wieder auf etwa 200 bis 250°C aufheizt und erneut besprüht. Beim Einsatz stark poröser anorganischer Grundmassen ist es vorteilhaft, die Besprühung bei Temperaturen im Bereich 250 bis 300°C durchzuführen. Hierdurch kann das Eindringen der kieselsäurehaltigen Masse in die anorganische Grundmasse auf ein Mindestmaß zurückgedrängt werden. Beim Einsatz stark poröser anorganischer Grundmassen kann die Anwendung höherer Temperaturen auch dadurch erforderlich werden, weil die oben angegebenen Grenzen für den Wasseranteil sonst nicht eingehalten werden können.

Beim Aufbringen der Kieselsäure enthaltenden Schicht auf eine organische Grundmasse kann man beispielsweise so verfahren, daß man die Grundmasse in einer heizbaren Dragiertrommel auf eine Temperatur erhitzt, die etwa 5 bis 15°C unterhalb des Erweichungspunktes der Grundmasse liegt. Falls man hierbei bei Temperaturen unter 100°C arbeiten muß, sprüht man dann nur kurz die Kieselsäure enthaltende Masse auf, läßt das Wasser verdunsten, heizt die Schüttung wieder auf die Ausgangstemperatur und besprüht erneut mit der Kieselsäure enthaltenden Masse. Die Temperatur der Schüttung sollte hierbei vorzugsweise nicht unter 60°C absinken. Sobald sich so viel Kieselsäure enthaltende Masse auf der organischen Grundmasse niedergeschlagen hat, daß diese ohne eintretende Verformung auf Temperaturen über 100°C erhitzt werden kann, führt man die Besprühung bei Temperaturen über 100°C durch, beispielsweise bei Temperaturen im Bereich 110 bis 220°C. Wenn der Erweichungspunkt der organischen Grundmasse über 110°C liegt, kann man beim Aufbringen der Kieselsäure enthaltenden Masse von Anfang an so verfahren, wie vorstehend beim Einsatz anorganischer Grundmassen beschrieben, allerdings ohne dabei so hohe Temperaturen anzuwenden, daß sich die Grundmasse verformt. Die maximale Temperatur, bei der organische Grundmassen beschichtet werden können, hängt von der Art der organischen Grundmasse ab. Im allgemeinen ist es vorteilhaft, Temperaturen von über 220°C zu vermeiden.

Das Aufbringen der kieselsäurehaltigen Schicht kann so erfolgen, daß bei einem Arbeitsgang eine Schicht beliebiger mittlerer Dicke, beispielsweise im Bereich von 10 bis 3000 µm, auf der Grundmasse entsteht. Man kann jedoch auch so verfahren, daß bei einem Arbeitsgang nur eine dünne Schicht, beispielsweise im Bereich einer mittleren Dicke von 1 bis 100 µm, entsteht und gegebenenfalls mehrere derartige Schichten nacheinander aufgebracht werden. Man kann auch mehrere dickere Schichten, beispielsweise solche mit einer mittleren Dicke im Bereich von 100 bis 1000 µm, nacheinander aufbringen. Wenn man eine dickere Schicht auf die Grundmasse aufbringen will, beispielsweise eine Schicht mit einer mittleren Schichtdicke über 100 µm, so ist es im allgemeinen vorteilhaft, diese Schicht in mehreren Arbeitsgängen durch Übereinanderlagern mehrerer dünnerer Schichten zu erzeugen, beispielsweise so, daß man eine Schicht mit einer mittleren Dicke von 3000 µm entweder in drei Arbeitsgängen herstellt, wobei je Arbeitsgang eine Schicht mit einer mittleren Dicke von etwa 1000 µm aufgebracht wird oder in 30 Arbeitsgängen, wobei je Arbeitsgang eine Schicht mit einer mittleren Dicke von ca. 100 µm aufgebracht wird.

Wen die kieselsäurehaltige Schicht in mehreren Arbeitsgängen auf die Grundmasse aufgebracht wird, so können zwischen den einzelnen Arbeitsgängen beliebig lange Pausen eingelegt werden. Es ist auch möglich, für die einzelnen Arbeitsgänge unterschiedlich zusammengesetzte Gemische zu verwenden, d. h., mehrere Schichten mit voneinander abweichender Zusammensetzung nacheinander aufzubringen. So ist es in manchen Fällen vorteilhaft, beispielsweise beim Einsatz von stark porösen Grundmassen oder solche mit sehr glatten Oberflächen (z. B. Stahlkugeln), zuerst eine Schicht aus einem Gemisch, das nur Wasser und Kieselsol und/oder Wasserglas enthält, zu erzeugen, wodurch die Porosität der Grundmasse herabgesetzt und/oder deren Haftfähigkeit für weitere Schichten verbessert wird, und danach eine oder mehrere Schichten aus Gemischen aufzubringen, die auch feinpulvrige, wasserunlösliche Kieselsäure, Porosierungsmittel und gegebenenfalls katalytische aktive Substanzen oder Vorläufer davon enthalten.

Die Dicke der in einem Arbeitsgang aufgebrachten Schicht hängt im wesentlichen vom Wassergehalt des aufzubringenden Gemisches und der Zeit ab, während der die gegebenenfalls schon mit einer oder mehreren kieselsäurehaltigen Schichten überzogene Grundmasse mit aufzubringendem Gemisch in Kontakt gebracht wird. Dem Fachmann bereitet es keine Schwierigkeiten, durch Variation dieser beiden Parameter Schichten von der gewünschten Dicke aufzubringen.

Die mittlere Dicke einer einzelnen Schicht beträgt vorzugsweise mindestens 10 µm, besonders bevorzugt mindestens 20 µm.

Die Dicke einer Schicht kann, im wesentlichen bedingt durch die Aufbringverfahren, sowohl von Partikel zu Partikel als auch innerhalb eines Partikels variieren. Dies ist jedoch im allgemeinen ohne nachteiligen Einfluß auf die Verwendbarkeit der erfindungsgemäß hergestellten Formkörper als Katalysatoren oder Katalysatorträger. Beim Einsatz von unregelmäßig geformten Grundmassen, z. B. Mahl-

8

gut, Aschen oder Schlacken, tritt im allgemeinen beim Aufbringen einer oder mehrerer Schichten eine Annäherung an die Kugelform auf, weil an Kanten und stark konvex gekrümmten Bereichen der Partikel die aufgebrachte Schicht relativ dünn, dagegen bei stark konkav gekrümmten Bereichen der Partikel relativ dick ist. Dies ist ebenfalls im allgemeinen ohne negativen Einfluß auf die Verwendbarkeit der erfindungsgemäß hergestellten Formkörper als Katalysatoren oder Katalysatorträger.

Nach dem Aufbringen von Kieselsäure auf eine anorganische Grundmasse kann das so erhaltene Produkt gegebenenfalls thermisch nachbehandelt werden. Dabei können die verschiedensten Temperaturen angewendet werden, beispielsweise solche im Bereich von 200 bis 1100°C. Bevorzugt sind dabei Temperaturen im Bereich 200 bis 700°C, besonders bevorzugt solche im Bereich 200 bis 500°C, ganz besonders bevorzugt solche im Bereich 200 bis 300°C. Eine thermische Nachbehandlung ist insbesondere dann vorteilhaft, wenn Porosierungsmittel eingesetzt worden sind.

Nach dem Aufbringen von Kieselsäure auf eine organische Grundmasse ist in jedem Fall eine thermische Nachbehandlung bei Temperaturen im Bereich 200 bis 1300°C erforderlich, um die organische Grundmasse zu entfernen. Bevorzugt sind hier Temperaturen im Bereich 300 bis 700°C. Im allgemeinen geht man so vor, daß die organische Grundmasse langsam über Schmelzen, Zersetzen, Verkohlen und stille Verbrennung entfernt wird, beispielsweise, indem man die Temperatur von 200 auf 700°C mit einer Steigerungsrate von 30 bis 70°C pro Stunde erhöht und dann so lange auf 700°C hält, bis der gesamte Restkohlenstoff, erkennbar an der schwarzen Verfärbung der kieselsäurehaltigen Schicht, verbrannt ist. Auf diese Weise erhält man Hohlräume, die von einer poröse Kieselsäure enthaltenden Schicht umgeben sind. Die organische Grundmasse kann auch erst dann entfernt werden, wenn in die Kieselsäure enthaltende Schicht katalytisch aktive Materialien oder Vorläufer davon eingebracht worden sind.

Wenn man erfindungsgemäße kieselsäurehaltige Formkörper herstellen will, welche katalytisch aktive Substanzen oder Vorläufer davon enthalten, so kann man katalytisch aktive Substanzen oder Vorläufer davon, z. B. der vorbeschriebenen Art, zusammen mit der Kieselsäure enthaltenden Masse auf die Grundmasse aufbringen. Dabei sind gegebenenfalls die Temperaturen während und nach dem Aufbringen der Kieselsäure enthaltenden Schicht(en) so zu kontrollieren, daß keine unerwünschten Veränderungen der katalytisch wirksamen Substanzen oder der Vorläufer davon eintreten. Man kann jedoch auch so verfahren, daß man zunächst auf die Grundmasse eine Masse aufbringt, die eine von katalytisch aktiven Substanzen oder Vorläufern davon freie kieselsäurehaltige Schicht ergibt und anschließend, beispielsweise durch übliche Tränk- oder Sprühverfahren, Lösungen oder Suspensionen von katalytisch aktiven Substanzen oder Vorläufern davon in die kieselsäurehaltige Schicht einbringt.

Unabhängig davon, wie die katalytisch aktiven Substanzen oder deren Vorläufer eingebracht worden sind, können gegebenenfalls die verschiedensten Nachbehandlungen vorgenommen werden, um aktive oder besser aktive Katalysatoren zu erhalten. Diese Nachbehandlung kann beispielsweise aus einer Wärmebehandlung, einer Reduktion oder einer Oxidation bestehen.

Die erfindungsgemäßen Produkte zeichnen sich dadurch aus, daß eine poröse Kieselsäure enthaltende Schicht beliebiger Dicke einen Hohlraum oder eine beliebige anorganische Grundmasse umgibt. Werden diese Produkte wie vorstehend beschrieben hergestellt, so erhält man eine feste kieselsäurehaltige Schichten. Die erfindungsgemäßen Produkte können, sofern sie keine katalytisch aktiven Zentren enthalten, als Katalysatorträgermaterial verwendet werden. Man kann daraus die verschiedensten Katalysatoren herstellen, die dann alle den Vorteil haben, daß die katalytisch aktiven Bestandteile nur in der kieselsäurehaltigen Schicht vorliegen, was sehr aktive und selektive Katalysatoren ergibt und wobei katalytisch aktives Material eingespart werden kann, da das Innere dieser Katalysatoren frei davon ist. Werden zusammen oder nach dem Aufbringen der Kieselsäure enthaltenden Schicht(en) katalytisch aktive Substanzen eingebracht oder erzeugt, werden diese Vorteile ebenfalls erhalten. Im Gegensatz zu den bekannten Katalysatoren, die eine TiO₂-haltige Schicht aufweisen, weisen die erfindungsgemäßen Produkte die Vorteile auf, daß sie sowohl an der Oberfläche als auch innerhalb der aufgebrachten Schicht(en) porös und hinsichtlich der katalytisch aktiven Bestandteile nicht auf solche beschränkt sind, die niedrige Schmelzpunkte haben. Besonders vorteilhaft hat sich die Beständigkeit der kieselsäurehaltigen Schicht gegenüber eingearbeiteten katalytisch aktiven Komponenten mit saurem Charakter und gegenüber sauren Reaktanten erwiesen, wogegen TiO₂-haltige Schichten unter diesen Bedingungen aufgrund einer chemischen Veränderung des TiO₂ instabil bis zum Zerfall sind.

Die erfindungsgemäßen kieselsäurehaltigen Formkörper, die katalytisch aktive Substanzen enthalten, können als Katalysatoren verwendet werden.

Beispielsweise kann man aus einem erfindungsgemäßen Produkt, das keine katalytisch aktiven Bestandteile enthält, einen hervorragenden Hydrierkatalysator erhalten, indem man eine Palladiumsalzlösung durch Tränkung aufbringt, z. B. eine wäßrige Palladiumchloridlösung, danach trocknet und das Palladiumsalz reduziert. Ein so hergestellter Katalysator ist beispielsweise für die Hydrierung von Nitroverbindungen besonders geeignet.

Wenn man Metallsalz- und/oder Edelmetallsalzlösungen auf erfindungsgemäße, von katalytisch aktiven Bestandteilen freie Produkte auftränken will, so können beispielsweise eines oder mehrere Metalle, beispielsweise gelöst in Form ihrer Salze, aufgetränkt werden. Physikalische Parameter, wie

9

z. B. Saugfähigkeit des Trägers und Löslichkeit der Metall- und/oder Edelmetallsalze, können zur Erreichung der geforderten Wirkstoffkonzentration im fertigen Katalysator Mehrfachtränkungen erforderlich machen. Beispielsweise wird die Konzentration der Metallsalz- und/oder Edelmetallsalzlösungen so eingestellt, daß der fertige Katalysator 0,5 bis 200 g, bevorzugt 1 bis 100 g einer oder mehrerer katalytisch wirksamer Komponenten pro Liter Träger enthält. Sofern die katalytisch wirksame Komponente ein Edelmetall oder eine Edelmetallverbindung darstellt oder sofern mehrere katalytisch aktive Komponenten in dem Trägerkatalysator vorliegen, von denen mindestens eine ein Edelmetall oder eine Edelmetallverbindung darstellt, kann der Gehalt dieser Komponenten beispielsweise jeweils 0,5 bis 100 g, bevorzugt 1 bis 50 g, besonders bevorzugt 2 bis 20 g betragen, berechnet als Edelmetall in elementarer Form pro Liter Träger.

Beispielsweise kann ein solcher Katalysator 1 bis 20 g, bevorzugt 2 bis 10 g Palladium oder 1 bis 100 g, bevorzugt 2 bis 50 g, eines Nicht-Edelmetalls oder, im Fall eines Mehrkomponenten-Trägerkatalysators, 1 bis 20 g Palladium, 1 bis 50 g eines ersten Übergangselementes und 1 bis 50 g eines zweiten Übergangselementes, jeweils berechnet als Metall in elementarer Form, pro Liter Träger enthalten.

Eine technische Durchführungsform dieser Tränkung sei anhand der Herstellung eines palladiumhaltigen SiO$_2$-Steatit-Katalysators veranschaulicht:

Erfindungsgemäß wird aus Steatit als anorganischer Grundmasse ein eine Kieselsäureschicht enthaltender Träger hergestellt, der entsprechend seiner Saugfähigkeit mit einer Metallsalzlösung, z. B. Natriumpalladiumchlorid, getränkt und getrocknet wird. Falls erforderlich, wird das aufgebrachte Metallsalz nach bekannten Methoden, beispielsweise Behandlung mit Hydrazinhydrat-Lösung, zuerst zu Metall reduziert, bevor sich ein Waschprozeß und eine Trocknung anschließen.

In dem so hergestellten Katalysator befindet sich das Palladium ausschließlich in der kieselsäurehaltigen Schicht. Die so hergestellten Trägerkatalysatoren können für die verschiedensten katalytischen Verfahren, z. B. Hydrierung, Dehydrierung, Hydrogenolyse, Oxidation, Acetoxidation, Polymerisation, Isomerisierung oder Cyclisierung, verwendet werden. Bei diesen katalytischen Reaktionen können so hergestellte Trägerkatalysatoren sowohl beim Arbeiten in der Sumpfphase als auch beim Arbeiten in der Rieselphase oder in der Gasphase eingesetzt werden. Bevorzugt sind die Rieselphase und die Gasphase. Dabei kann bei Normaldruck, Überdruck oder vermindertem Druck gearbeitet werden.

Katalytische Hydrierungen sind ein bevorzugtes Anwendungsgebiet für so hergestellte Katalysatoren. Je nach Wirkstoffzusammensetzung eignen sie sich besonders zur Hydrierung aliphatischer Mehrfachbindungen, z. B. für Selektivhydrierungen, zur Kernhydrierung aromatischer Systeme oder zur Hydrierung bestimmter Substituenten, beispielsweise von in aromatischen Systemen enthaltenen Nitro- oder Carbonylgruppen. Bestimmte Wirkstoffzusammensetzungen der so hergestellten Trägerkatalysatoren haben eine bevorzugte Anwendung bei katalytischen Hydrierungen, z. B. von substituierten Aromaten, wobei, abhängig von der Kombination der katalytisch wirksamen Substanzen sowie anderer Verfahrensparameter, wie Temperatur oder Druck, entweder das aromatische System und/oder der Substituent hydriert werden können.

Ein weiteres wichtiges Einsatzgebiet ist die Acetoxidation von Olefinen, z. B. die Bildung von Vinylacetat aus Ethylen, Essigsäure und Sauerstoff unter Einsatz von erfindungsgemäß hergestellten Katalysatoren, die Palladium, Gold und Alkaliacetat als katalytisch aktive Substanzen enthalten.

Nach dem erfindungsgemäßen Verfahren hergestellte Katalysatoren mit der Wirksubstanz Palladium auf SiO$_2$-Steatit finden eine bevorzugte Anwendung zur katalytischen Hydrierung von Nitroaromaten, z. B. von Nitrobenzol, Nitrotoluol, Dinitrobenzolen, Dinitrotoluolen, Trinitrotoluolen, Nitrophenolen und Nitrochloraromaten zu den entsprechenden aromatischen Aminen. Für Mononitroverbindungen wird dabei die Gasphasenreaktion bevorzugt, während für die Dinitro- oder Trinitroverbindungen die Flüssigphase und speziell die Rieselphase bevorzugt wird. Sowohl in der Gasphase als auch in der Rieselphase wird im allgemeinen die zu reduzierende Verbindung über einen fest angeordneten Kontakt geleitet. Es wird vorteilhaft mit einem Überschuß an Wasserstoff gearbeitet. Beim Arbeiten in der Rieselphase wird die zu reduzierende Nitroverbindung gewöhnlich mit der bei der Reduktion entstehenden Aminoverbindung oder einem anderen Verdünnungsmittel so weit verdünnt, daß eine Durchführung der Reduktion ungefährlich ist. Die bevorzugte Reaktionstemperatur in der Rieselphase liegt im Bereich von 50 bis 150° C und der bevorzugte Druckbereich zwischen 1 und 100 bar.

Bei der Hydrierung in der Gasphase wird bevorzugt im Temperaturbereich von 150 bis 350° C und bei 1 bis 10 bar gearbeitet.

Ganz allgemein ist festzustellen, daß die Anwendung der erfindungsgemäßen Produkte als Katalysatoren und Katalysatorträger praktisch nicht beschränkt ist. Man kann in die kieselsäurehaltige Schicht der erfindungsgemäßen Produkte praktisch jede für eine heterogene und/oder homogene Katalyse geeignete, katalytisch aktive Substanz einbringen oder darin erzeugen und die so erhaltenen Katalysatoren dann in Verfahren einsetzen, in denen die Wirksamkeit der jeweiligen katalytisch aktiven Substanzen bekannt ist. Die Verfahrensbedingungen derartiger katalytischer Verfahren (Einsatzmengen, Druck, Temperatur, Verweilzeit etc.) ändern sich beim Einsatz der erfindungsgemäßen Katalysatoren im allgemeinen nur in dem Ausmaß, wie es aufgrund der verbesserten Aktivität und/oder Selektivität der erfindungsgemäßen Katalysatoren möglich ist. Man kann auch in vielen Fällen einfach erfindungsgemäße Katalysatoren mit geringerer Wirkstoffmenge als bisher einsetzen und bei unveränderten Verfahrensbedingungen gleiche Ergebnisse wie bisher erhalten.

Als Beispiele für die Anwendung erfindungsgemäß zugänglicher Katalysatoren seien folgende angeführt:

a) Katalysatoren für Hydrierungen, die Edelmetalle und/oder sonstige Metalle enthalten, im einzelnen z. B. folgende:
Für die Hydrierung von Dreifach- zu Doppelbindungen Katalysatoren, die Palladium oder Palladium mit Zusätzen von Blei, Zink, Kupfer, Chrom oder Aminen und Schwefelverbindungen enthalten. Für die Hydrierung von Diolefinen zu Monoolefinen Katalysatoren, die Palladium enthalten. Für die Hydrierung von Monoolefinen, z. B. bei der Fetthärtung, Katalysatoren, die Raney-Nickel enthalten. Für die Hydrierung ungesättigter Aldehyde zu gesättigten Aldehyden Katalysatoren, die Platinmetalle enthalten. Für die Hydrierung von ungesättigten Nitrilen zu gesättigten Nitrilen Katalysatoren, die Palladium enthalten. Für die Kernhydrierung von Aromaten Katalysatoren, die Raney-Nickel oder Rhodium enthalten. Für die Kernhydrierung von Phenolen zu Cyclohexanolen Katalysatoren, die Palladium enthalten. Für die Hydrierung von Phenol zu Cyclohexanon Katalysatoren, die Platin enthalten. Für die Hydrierung von Aldehyden zu Alkoholen Katalysatoren, die Nickel enthalten. Für die Hydrierung von ungesättigten Aldehyden zu ungesättigten Alkoholen Katalysatoren, die Platin, gegebenenfalls mit Zusätzen von Zink und/oder Eisen, enthalten. Für die Hydrierung von Carbonsäuren zu Alkoholen Katalysatoren, die Raney-Kobalt enthalten. Für die Hydrierung von Carbonsäureanhydriden zu Lactonen oder Diolen Katalysatoren, die Nickel enthalten. Für die Hydrierung von Säurechloriden zu Aldehyden Katalysatoren, die Palladium und schwefelhaltige Verbindungen enthalten. Für die Hydrierung von Nitrilen zu Aminen Katalysatoren, die entweder Raney-Kobalt oder Eisen mit Mangan und phosphorhaltigen Zusätzen enthalten. Für die Hydrierung von ungesättigten Nitrilen zu ungesättigten Aminen Katalysatoren, die Kupfer-Chromit und Zusätze von Bariumverbindungen enthalten. Für die Hydrierung von Aldoximen zu Hydroxylaminen Katalysatoren, die Palladium oder Platin enthalten. Für die Hydrierung von Aldoximen zu Aminen Katalysatoren, die Rhodium oder Platin enthalten. Für die Umsetzung von Ketonen mit Wasserstoff und Ammoniak zu Aminen Katalysatoren, die Kobalt und Nickel enthalten. Für die Umsetzung von Alkoholen mit Ammoniak und/oder Aminen und Wasserstoff zu Aminen Katalysatoren, die Nickel enthalten. Für die Hydrierung von Nitroverbindungen zu Aminen Katalysatoren, die Kupfer-Chromit und Bariumverbindungen oder Palladium enthalten. Für die Ammoniak-Synthese Katalysatoren, die Eisen mit Zusätzen von Aluminium-, Kalium-, Magnesium- und Siliciumverbindungen enthalten.

b) Katalysatoren für Dehydrierungen und oxidative Dehydrierung, die Metallverbindungen enthalten, im einzelnen z. B. folgende:
Für die oxidative Dehydrierung von Olefinen zu Diolefinen, insbesondere die Umwandlung von Butenen zu Butadien, Katalysatoren, die Magnesium-Ferrit mit Phosphor und Nickel enthaltenden Zusätzen oder Calcium-Nickel-Phosphat und gegebenenfalls Strontium und/oder Chrom enthaltende Zusätze oder gemischte Molybdate (z. B. Nickel-Kobalt-Eisen-Bismut-Molybdat und gegebenenfalls Phosphor und Kalium enthaltende Zusätze) oder Nickel-Calcium-Phosphat oder gemischte Antimon-Zinn-Oxide enthalten. Für die Dehydrierung von Kohlenwasserstoffen, insbesondere von Ethylbenzol zu Styrol, Katalysatoren, die Eisenoxid und Zusätze von Chromoxid und Kaliumverbindungen enthalten. Für die Dehydrierung von linearen Kohlenwasserstoffen zu Aromaten, insbesondere von n-Hexan zu Benzol, Katalysatoren, die Chromoxid und Aluminiumoxid mit Zusätzen von Alkaliverbindungen enthalten. Für die Umwandlung von Cyclohexanol zu Cyclohexanon Katalysatoren, die Zinkoxid und Zusätze von Alkali- und Erdalkaliverbindungen enthalten.

c) Katalysatoren für die Hydratisierung oder Dehydratisierung, die Phosphorverbindungen und/oder Zeolithe enthalten, im einzelnen z. B. folgende:
Für die Hydratisierung von Olefinen zu Alkoholen, z. B. von Ethylen zu Ethylalkohol, Katalysatoren, die Phosphorsäure und Siliciumdioxid enthalten. Für die Dehydratisierung von sekundären Alkoholen zu Olefinen, insbesondere von $\beta$-Hydroxyethylbenzol zu Styrol, Katalysatoren, die Natriummetaphosphat und Siliciumdioxid oder Zeolithe oder Aluminiumoxid enthalten. Für die Umsetzung von Carbonsäuren mit Ammoniak unter Wasserabspaltung zu Nitrilen, insbesondere von Adipinsäure zu Adipinsäuredinitril, Katalysatoren, die Phosphorsäure und Siliciumdioxid enthalten. Für die Dehydratisierung von 1,4-Butandiol zu Tetrahydrofuran Katalysatoren, die saure Zeolithe enthalten.

d) Katalysatoren für Umsetzungen in Gegenwart von Kohlenmonoxid, die Übergangsmetalle oder Übergangsmetallverbindungen enthalten, im einzelnen z. B. folgende:
Für die Synthese von Alkoholen, insbesondere Methanol, aus Kohlenmonoxid und Wasserstoff Katalysatoren, die entweder Zinkoxid oder Chromoxid oder Kupfer und Zusätze von Zinkoxid, Aluminiumoxid, Chromoxid und/oder Alkaliverbindungen enthalten. Für die Fischer-Tropsch-Synthese Katalysatoren, die Eisen und Zusätze von Alkaliverbindungen, Thoriumoxid und Magnesiumoxid enthalten oder Katalysatoren, die Eisen und Aluminiumoxid enthalten. Für die Methanisierung Katalysatoren, die Nickel und/oder Aluminiumoxid oder Spinelle enthalten oder Katalysato-

11

ren, die Ruthenium enthalten. Für die Umsetzung von Kohlenmonoxid mit Wasserdampf (Konvertierung) Katalysatoren, die Eisen- und Chromoxid oder Kupfer, Zinkoxid und Aluminiumoxid oder Kobalt-Molybdän-Sulfid und Aluminiumoxid enthalten. Für das Steam-Reforming Katalysatoren, die Nickel und Alkaliverbindungen enthalten.

e) Katalysatoren für die Ammonoxidation, die Übergangsmetalloxide enthalten, im einzelnen z. B. folgende:
Für die Umsetzung von Propylen zu Acrylnitril Katalysatoren, die Vanadium-Antimon-Oxide oder Molybdän-Bismut-Eisen-Kobalt-(Nickel-)Oxide und Zusätze von Kalium- und Phosphorverbindungen oder Antimon-Eisen-Oxide enthalten. Für die Umsetzung von o-Xylol zu Phthalodinitril Katalysatoren, die Vanadium-Antimon-Oxide enthalten.

f) Katalysatoren für Oxidationsreaktionen, die Übergangsmetalle oder Übergangsmetalloxide enthalten, im einzelnen z. B. folgende:
Für die Umsetzung von Olefinen zu ungesättigten Aldehyden, insbesondere von Propylen zu Acrolein und i-Buten zu Methacrolein, Katalysatoren, die Kupferoxid oder Molybdän-Bismut-Eisen-Kobalt-(Nickel-)Phosphor-Oxide oder Molybdän-Niob-(Tantal-)Bismut-Oxide, jeweils gegebenenfalls mit Promotoren, enthalten. Für die Umsetzung von ungesättigten Aldehyden zu ungesättigten Säuren, insbesondere von Acrolein zu Acrylsäure und Methacrolein zu Methacrylsäure, Katalysatoren, die Molybdän-Vanadium-Wolfram-Oxide oder Molybdän-Phosphor-Niob-(Tantal-)Oxide, jeweils gegebenenfalls mit Promotoren, enthalten. Für die Umsetzung von $C_4$-Kohlenwasserstoffen zu Maleinsäureanhydrid Katalysatoren, die Vanadium- und Phosphoroxid oder Molybdän-Antimon-Vanadium-Oxide, jeweils gegebenenfalls mit Promotoren, enthalten. Für Oxiacetylierungen von Olefinen unter Bildung von Estern ungesättigter Alkohole (z. B. Vinylacetat und Allylacetat) Katalysatoren, die Palladium und Alkaliacetate und gegebenenfalls weitere Metalle oder Metallverbindungen (z. B. Gold, Bismut, Cadmium, Mangan) enthalten. Für die Oxidation von Ethylen zu Ethylenoxid Katalysatoren, die Silber und gegebenenfalls Zusätze von Alkaliverbindungen enthalten. Für die Oxidation von Alkoholen zu Aldehyden, insbesondere Methanol zu Formaldehyd, Katalysatoren, die Silber oder Molybdän-Eisen-Oxide enthalten. Für die Oxidation von Kohlenwasserstoffen zu Carbonsäuren oder Aldehyden, insbesondere von n-Buten zu Essigsäure, Katalysatoren, die Titan-Vanadat enthalten. Für die Oxidation von Aromaten zu Säureanhydriden oder Chinonen, z. B. von Benzol, Naphthalin oder o-Xylol zu Phthalsäureanhydrid, und gegebenenfalls Naphthochinon Katalysatoren, die Vanadiumpentoxid mit Zusätzen von Molybdän-/Phosphoroxiden oder Siliciumdioxid/Kaliumpyrosulfat enthalten, und für die Oxidation von Anthracen zu Anthrachinon Katalysatoren, die Vanadiumpentoxid enthalten.

g) Katalysatoren für die Gasreinigung, die Metalle oder Metalloxide enthalten, im einzelnen z. B. folgende:
Für die Entgiftung von Gasen aus Verbrennungsmaschinen Katalysatoren, die Platin und Ruthenium oder Platin und Palladium oder Platin und Rhodium, jeweils gegebenenfalls mit Promotoren, enthalten. Für die Entfernung von Schwefelwasserstoff nach dem Claus-Verfahren Katalysatoren, die aktives Aluminiumoxid enthalten. Für die Entfernung kleiner Mengen Chlor oder Schwefel Katalysatoren, die Kupfer oder Zinkoxid enthalten. Für die Entfernung kleiner Kohlenmonoxid- oder Kohlendioxid-Mengen Katalysatoren, die Nickel und Magnesium-Aluminium-Spinell oder Ruthenium enthalten.

Die folgenden Beispiele erläutern die vorliegende Erfindung ohne sie in irgendeiner Weise zu beschränken.

**0 083 791**

Beispiele

Übersicht

Teil A

Beschreibung der Einsatzmaterialien

Teil B

Beschreibung von Herstellungsmethoden für erfindungsgemäße Produkte

Beispiel 1 Sprühverfahren — Einmalbeschichtung
2 Sprühverfahren — Mehrfachbeschichtung
3 Thermische Nachbehandlung

Teil C

Vergleiche erfindungsgemäßer Katalysatoren mit dem Stand der Technik

Beispiel 1 Acetoxidation, Vinylacetatherstellung
2 Oxidation von Naphthalin
3 Hydrierung von o-Nitrotoluol

Teil D

Herstellungsbeispiele für erfindungsgemäße Produkte

Beispiele 1—29

Bei Prozentangaben handelt es sich, soweit nichts anderes angegeben ist, um Gewichtsprozente.

**0 083 791**

Teil A

Beschreibung der Einsatzmaterialien

Bei den Materialien 1 bis 8 handelt es sich um verschiedene anorganische Grundmassen, bei den Materialien 9 bis 17 um sonstige Einsatzmaterialien für die in den Teilen B, C und D beschriebenen Versuche.

| | Material-Nr. | | | |
| --- | --- | --- | --- | --- |
| | 1 Steatit | 2 Steatit | 3 Asche | 4 $SiO_2$ |
| Form | Kugeln 3,0—3,5 mm | Hohl-Zylinder 7 mm Außen-durchmesser | Mahlgut 2,8—3,5 mm | Kugeln 4,7—5,8 mm |
| Chem. Zusammensetzung [%] | | | | |
| $SiO_2$ | | 62,15 | 41,30 | 91,79 |
| $Al_2O_3$ | | 4,28 | 22,58 | 3,09 |
| $Fe_2O_3$ | | 2,23 | 16,38 | 0,58 |
| $Mn_2O_3$ | | — | 0,10 | — |
| $TiO_2$ | | 0,16 | 0,92 | 0,45 |
| CaO | | — | 7,72 | — |
| MgO | | 29,88 | 3,80 | 0,13 |
| $K_2O$ | | 0,65 | 3,50 | 0,77 |
| $Na_2O$ | | 0,93 | 1,10 | — |
| Physikalische Daten | | | | |
| Schüttgewicht [kg/l] | 1,615 | 1,070 | 1,250 | 0,540—0,590 |
| Saugfähigkeit [g $H_2O$/100 g Material] | — | — | — | 63 |
| BET-Oberfläche [$m^2$/g] | <1 | <1 | <0,1 | 150 |
| Wahre Dichte [g/$cm^3$] | | | 2,732 | |
| Scheinbare Dichte [g/$cm^3$] | | | 2,529 | |
| Hg-Porenvol. [$mm^3$/g] | | | 15 | |
| Gesamtporenvol. [$mm^3$/g] | | | 29 | 750 |
| Porosität [%] | | | 7,4 | 64 |

(Fortsetzung)

| | Material-Nr. | | | |
|---|---|---|---|---|
| | 5<br>Stahl | 6<br>Edelstahl | 7<br>Silicium-<br>carbid | 8<br>Al$_2$O$_3$ |
| Form | Kugeln<br>3,5 mm | Raschig-<br>ringe<br>5 × 5 mm | Kugeln<br>5 mm | Kugeln<br>5 mm |
| **Chem. Zusammensetzung [%]** | | | | |
| SiO$_2$ | | | 28,5 | <0,1 |
| Al$_2$O$_3$ | | | 4,7 | >95 |
| Fe$_2$O$_3$ | | | 0,3 | <0,1 |
| Mn$_2$O$_3$ | | | — | |
| TiO$_2$ | | | <0,01 | |
| CaO | | | 0,2 | |
| MgO | | | 0,1 | |
| K$_2$O | | | 0,1 | |
| Na$_2$O | | | 0,1 | 0,7 |
| **Physikalische Daten** | | | | |
| Schüttgewicht [kg/l] | 4,722 | 0,200 | 1,000 | 0,760 |
| Saugfähigkeit [g H$_2$O/100 g Material] | — | — | 20,45 | 45 |
| BET-Oberfläche [m$^2$/g] | — | — | <0,3 | 350 |
| Wahre Dichte [g/cm$^3$] | | | | |
| Scheinbare Dichte [g/cm$^3$] | | | | |
| Hg-Porenvol. [mm$^3$/g] | | | | 450 |
| Gesamtporenvol. [mm$^3$/g] | | | | |
| Porosität [%] | | | | |

Material Nr. 9

Kieselsol

Typ Bayer Kieselsol 300, 30% (Handelsbezeichnung)

| | |
|---|---|
| $SiO_2$-Gehalt [%] | ca. 30 |
| $Na_2O$-Gehalt [%] | ca. 0,35 |
| pH-Wert | ca. 9,8 |
| Dichte [g/cm³] | 1,2 |
| Viskosität [mPa · sec] | 4−6 |
| Spez. Oberfläche [m²/g] | ca. 280−320 |
| Teilchengröße [nm] | 7−8 |
| Ionogenität | anionisch |
| Farbe | klar |

Material Nr. 10

Kieselsäure

Typ Ultrasil VN 2 (Handelsbezeichnung)

| | |
|---|---|
| Glühverlust bei 1000° C [%] | 11 |
| davon Feuchte bei 105° C [%] | 6 |
| $SiO_2$-Gehalt [%] | 87 |
| $Al_2O_3$-Gehalt [%] | 0,2 |
| $Na_2O$-Gehalt [%] | 0,8 |
| $SO_3$-Gehalt [%] | 0,5 |
| $Fe_2O_3$-Gehalt [%] | <0,05 |
| Spez. Gewicht [g/cm³] | 2,0 |
| Stampfgewicht [g/l] | 200 |
| pH-Wert | 7 |
| BET-Oberfläche [m²/g] | 130 |
| Mittlere Primärteilchengröße [nm] | 28 |

Material Nr. 11

Wasserglas

Typ Kaliumsilikat als wäßrige Lösung

| | |
|---|---|
| Dichte [g/cm³] | 1,25 |
| $SiO_2$ [%] | 21 |
| $K_2O$ [%] | 8,1 |

Material Nr. 12

Kieselgur

Typ gereinigt und geglüht nach DAB Erg. B 6

| | |
|---|---|
| $SiO_2$ [%] | >96 |
| pH-Wert | 5−9 |
| In Salzsäure lösliche Anteile [%] | <1 |
| Säurelösliches Sulfat [%] | <0,02 |
| Säurelösliches Eisen [%] | <0,04 |
| Glühverlust (600° C) [%] | <0,5 |
| Siebrückstand >0,1 mm [%] | <2 |

16

Material Nr. 13

Vanadyloxalat-Lösung

Wäßrige Lösung mit einem Gehalt an Vanadyloxalat von 17,8 Gew.-% entsprechend 10,4 Gew.-% $V_2O_5$ und einer Dichte von 1,165 g/cm$^3$.

Material Nr. 14

Aktiv-Kohle

Bayer Carboraffin P (Handelsbezeichnung)

| | |
|---|---|
| Wasser bei Abpackung [Gew.-%] | $<10$ |
| Mahlfeinheit [Gew.-%] | $20-75\,\mu m$ |
| Rütteldichte [kg/l] | ca. 0,3 |
| pH-Wert | $5-7$ |
| Asche [Gew.-%] | $2,0-3,0$ |
| Melasse-Faktor | ca. 1,0 |

Material Nr. 15

Zeolith

Bayer-Zeolith T-Pulver (Handelsbezeichnung) (Alkali-Aluminosilikat vom Typ A in der K-Form)

| | |
|---|---|
| Porenweite [nm] | 40 |
| Kristallitdurchmesser [$\mu$m] | $2-10$ |
| Schüttgewicht [g/l] | 350 |
| pH-Wert | 11 |

Material Nr. 16

Ionenaustauscher

Bayer Lewasorb SW 12 (Handelsbezeichnung)

| | |
|---|---|
| Lieferform | Na |
| Kornform | Mikrogranulat |
| Grundgerüst | Polystyrol |
| Ankergruppe | Sulfonsäure |
| Korngrößenbereich [nm] | $<0,1$ (95% $<0,075$) |
| Schüttgewicht [g/l] | $700-800$ |
| Dichte [g/cm$^3$] | 1,28 |

Material Nr. 17

Aluminiumoxid

Identisch mit Material Nr. 8, jedoch Korngröße $<0,1$ mm.

17

Material Nr. 18

Polystyrol

Granulat, ellipsenförmig bis 3 mm, Schüttgewicht 682 g/l, Erweichungstemperatur 75 bis 80°C, Handelsprodukt der BASF unter der Bezeichnung Polystyrol 143 c.

Material Nr. 19

Polymerlegierung aus Polycarbonat und ABS

Granulat, Strangstückchen bis 3 mm, Schüttgewicht 640 g/l, Erweichungstemperatur 120°C, Handelsprodukt der Bayer AG (s. Bayer Firmenschrift KL 46150 von 6.81).

Material Nr. 20

Polyethylen

Granulat, ellipsenförmig bis 3,5 mm, Schüttgewicht 638 g/l, Erweichungstemperatur 90°C, Handelsprodukt der Bayer AG (s. Bayer Firmenschrift KL 43570 von 6.82).

Material Nr. 21

Celluloseester

Granulat, Strangstückchen bis 3,1 mm, Schüttgewicht 754 g/l, Erweichungstemperatur 60 bis 110°C, Handelsprodukt der Bayer AG (s. Bayer Firmenschrift KL 40001 von 8.75).

Material Nr. 22

Glasfaserverstärktes Polyamid

Granulat, Strangstückchen bis 3,5 mm, Schüttgewicht 730 g/l, Erweichungstemperatur 255°C, 50% Glasfaseranteil, Handelsprodukt der Bayer AG (s. Bayer Firmenschrift KL 40360 von 4.78).

Material Nr. 23

Polycarbonat

Zylindrisches Granulat bis 3 mm, Schüttgewicht 773 g/l, Erweichungstemperatur bis 150°C, Handelsprodukt der Bayer AG (s. Bayer Firmenschrift KL 46100 von 8.79).

Material Nr. 24

Acrylnitril-Butadien-Styrol-Polymerisat (ABS-Polymerisat)

Kubisch geschnittenes Granulat bis 3,5 mm, Schüttgewicht 673 g/l, Erweichungstemperatur 102°C, Handelsprodukt der Bayer AG (s. Bayer Firmenschrift KL 41654 von 10.78).

Material Nr. 25

Glasfaserverstärkter Polyester

Granulat, Strangstückchen bis 3,8 mm, Schüttgewicht 864 g/l, Erweichungstemperatur bis 185°C, 30% Glasfaseranteil, Handelsprodukt der Bayer AG (s. Bayer Firmenschrift KL 41100 von 6.81).

Teil B

Beschreibung von Herstellungsmethoden für erfindungsgemäße Produkte
unter Verwendung anorganischer Grundmassen

Beispiel 1

Sprühverfahren — Einmalbeschichtung

In einer 50 l fassenden, heizbaren Dragiertrommel werden 1,615 g Material Nr. 1 (siehe Teil A) vorgelegt und bei einer Umdrehungsgeschwindigkeit von 3 U/min auf 200°C aufgeheizt.

Währenddessen werden in einem 3-l-Behälter 443 g Kieselsol, 133 g Ultrasil VN2 und 133 g Kieselgur (Materialien Nr. 9, 10 und 12, siehe Teil A) mit 1300 g Wasser zu einer 20gew.-%igen Suspension verrührt. Diese wird unter stetigem Rühren pneumatisch aus einem Druckbehälter heraus auf die ständig bewegte, 200°C heiße vorgelegte anorganische Grundmasse diskontinuierlich aufgesprüht.

Hierbei werden innerhalb von 15 Minuten die Umdrehungsgeschwindigkeit der Trommel von 3 auf 15 U/min gesteigert und 300 g Suspension aufgesprüht. Sobald die Temperatur des bewegten Materials 150°C unterschritten hat, wird der Sprühprozeß so lange unterbrochen, bis 200°C wieder erreicht sind. Die noch vorhandenen 1709 g der Suspension werden bei einer Umdrehungsgeschwindigkeit von 15 U/min diskontinuierlich innerhalb von 45 Minuten aufgetragen. Hierbei wird das bewegte Material innerhalb des Temperaturbereiches von 150 bis 200°C gehalten. Man läßt nach beendeter Auftragung das Produkt bei stillstehender Trommel auf 40°C abkühlen und entleert dann die Trommel.

Es werden 1765 g Produkt, enthaltend 150 g Kieselsäure, in Form einer Schicht mit einer mittleren Dicke von 175 μm erhalten. Die Saugfähigkeit dieses Produktes beträgt 4,2% bei einem Schüttgewicht von 1428 g/l und einer Restfeuchte von <1%.

Beispiel 2

Sprühverfahren — Mehrfachbeschichtung

Entsprechend Teil B, Beispiel 1, werden 4038 g Material Nr. 1 mit 4500 g einer aus 1000 g Kieselsol, 300 g Ultrasil VN2, 300 g Kieselgur (Materialien Nr. 9, 10 und 12, siehe Teil A) und 2900 g Wasser bestehenden Mischung innerhalb von 2 Stunden besprüht. Innerhalb der ersten 15 Minuten werden 400 g der Gesamtmischung aufgetragen und der Rest in weiteren 105 Minuten.

Es werden 4494 g Produkt, enthaltend 456 g Kieselsäure, in Form einer Schicht mit einer mittleren Dicke von 150 μm erhalten.

In einem weiteren Arbeitsgang werden 2085 g des Produktes erneut mit 4500 g der obenerwähnten Mischung unter den gleichen Bedingungen besprüht.

Es werden 2498 g Produkt, enthaltend 413 g Kieselsäure, in Form einer zweiten Schicht erhalten. Dieses Produkt besitzt nun insgesamt eine Kieselsäureschicht mit einer mittleren Gesamtdicke von 700 μm, ein Schüttgewicht von 1180 g/l und eine Saugfähigkeit von 11,9 ml/100 g.

Beispiel 3

Thermische Nachbehandlung

Das gemäß Teil B, Beispiel 2, nach einer zweimaligen Beschichtung erhaltene kieselsäurehaltige Produkt wurde einer thermischen Nachbehandlung unterzogen. Hierbei wurde das Produkt im Bereich von 200 bis 1100°C bei verschiedenen Temperaturen jeweils 3 Stunden thermisch, und zwar bis 500°C in der Dragiertrommel mittels eines Acetylen-Sauerstoff-Brenners und bis 1100°C in einer Glühmuffel behandelt. Hierbei konnten je nach der Temperatur die folgenden BET-Oberflächen eingestellt werden.

| | Temperatur [°C] | | | | | | | | | |
| | 200 | 300 | 400 | 500 | 600 | 700 | 800 | 900 | 1000 | 1100 |
|---|---|---|---|---|---|---|---|---|---|---|
| BET-Oberfläche, bezogen auf beschichtete Originalkugeln [m²/g] | 25,8 | 26,3 | 26,4 | 25,9 | 24,4 | 21,4 | 6,9 | 1,2 | 0,5 | 0,2 |

**0 083 791**

Gleiche Ergebnisse erhält man, wenn man die thermische Nachbehandlung von Anfang an in einer Glühmuffel vornimmt.

## Teil C

### Vergleiche erfindungsgemäßer Katalysatoren mit dem Stand der Technik

### Beispiel 1

### Acetoxidation, Vinylacetatherstellung

#### a) Vergleichskatalysator

In der DE-C-1 668 088 ist ein Katalysator für die Vinylacetatsynthese beschrieben. Eine Nacharbeitung der Beispiele dieser Patentschrift ergibt, daß im Katalysatorträger ein innerer Bereich ausgebildet ist, der eine Palladium/Gold-Legierung enthält, wogegen das Alkaliacetat durch den gesamten Träger verteilt ist. Der Katalysator liefert 245 g Vinylacetat pro Stunde und pro Liter Katalysator bei 140°C. Der Katalysator wird gemäß Beispiel 1 der DE-C-1 668 088 dadurch hergestellt, daß man den Träger gleichzeitig mit einer Lösung von Palladium- und Goldsalzen und einer Lösung behandelt, die Verbindungen enthält, welche zur Reaktion am Katalysatorträger mit den Palladium- und Goldsalzen unter Bildung wasserunlöslicher Palladium- und Goldverbindungen befähigt sind, anschließend die wasserunlöslichen Palladium- und Goldverbindungen durch Behandlung mit Reduktionsmitteln in die Edelmetalle überführt und schließlich die wasserlöslichen Verbindungen auswäscht. Danach wird ein Alkaliacetat (Kaliumacetat) durch Tränkung aufgebracht, so daß der fertige Katalysator ca. 30 g Kaliumacetat/l enthält.

#### b) Erfindungsgemäßer Katalysator

1180 g = 1000 ml des nach Teil B, Beispiel 2, durch Zweifachbeschichtung hergestellten Produktes werden mit 140 ml einer Lösung, die 3,3 g Pd als $Na_2PdCl_4$ und 1,5 g Au als $HAuCl_4$ enthält, imprägniert. Das Volumen der Tränklösung entspricht der Saugfähigkeit des Produktes. Das so imprägnierte Produkt wird zur Reduktion der Edelmetalle mit 5%iger Hydrazinhydratlösung überschichtet und 4 Stunden zur Vervollständigung der Reduktion stehengelassen. Danach wird das Produkt mit Wasser gewaschen und anschließend anhaftendes Wasser durch Trocknen entfernt. Man erhält so 1168,7 g eines Katalysators, der noch mit 135 ml einer wäßrigen Lösung, die 15 g Kaliumacetat enthält, erneut, entsprechend seiner Saugfähigkeit, getränkt wird. Nach einem Trockenprozeß bei 115°C erhält man 1180 g = 970 ml eines Katalysators, der zur Vinylacetatherstellung eingesetzt werden kann.

#### c) Vinylacetat-Herstellung

Unter den in der DE-C-1 668 088, Beispiel 5, beschriebenen Bedingungen werden 900 ml des gemäß b) hergestellten Katalysators in ein Reaktionsrohr von 25 mm Innendurchmesser und 2 m Länge eingefüllt. Über den Katalysator werden bei 140°C und einem Druck von 8 bar stündlich 77 Mol Ethylen, 19 Mol Essigsäure gasförmig und 5,8 Mol Sauerstoff geleitet. Es wurden pro Liter Katalysator stündlich 357 g Vinylacetat gebildet.

### Beispiel 2

### Oxidation von Naphthalin

#### a) Vergleichskatalysator

Es wurde der Katalysator A aus Beispiel 1 der DE-A-2 453 232 hergestellt.
Hierbei wird eine aus $V_2O_5$, $H_2SO_4$ und $SO_2$ erhaltene Vanadylsulfat-Lösung mit einer aus Kaliumsilikat-Lösung und Schwefelsäure hergestellten feinpulvrigen Kieselsäure vermischt. Die so erhaltene Paste wird in Lochbleche mit 5 mm Dicke und 5 mm Durchmesser gegeben und 2 h bei 50°C getrocknet. Die so erhaltenen Formkörper werden danach bei 125°C getrocknet und anschließend 12 Stunden bei 425°C getempert. Das ergab den Katalysator 1.

### b) Erfindungsgemäßer Katalysator

Entsprechend Teil B, Beispiel 1, werden 500 g Siliciumcarbid (siehe Teil A, Material Nr. 7), 253 g Kieselsol, 76 g Ultrasil VN2, 76 g Kieselgur (Materialien Nr. 9, 10 und 12, siehe Teil A), 106 g 97%ige Schwefelsäure, 301 g einer wäßrigen Vanadyloxalatlösung (entsprechend 31,8 g Vanadiumpentoxid) und 183 g Kaliumsulfat mit 215 g Wasser zu einer 46%igen Suspension vermischt. Diese Suspension wird entsprechend Teil B, Beispiel 1, auf das Siliciumcarbid aufgebracht.

Es werden 965 g beschichtetes Produkt erhalten mit einer Schichtdicke von 1000 μm. Das ergab den Katalysator 2.

### c) Naphthalin-Oxidation

Unter den in der DE-A-2 453 232 beschriebenen Bedingungen wurde Naphthalin mit Sauerstoff in Gegenwart des Katalysators 1 und separat davon in Gegenwart des Katalysators 2 umgesetzt. Die Umsetzungen wurden in einem mit einem Salzbad beheizten Reaktionsrohr aus Stahl von 3 m Länge und 30 mm innerem Durchmesser durchgeführt. In den Reaktor wurde zunächst bei Raumtemperatur bei einem Druck von 6 bar ein Gasgemisch aus 94% Stickstoff und 6% Sauerstoff mit einer Geschwindigkeit von 4 Nm³/h geleitet. Dieses Gasgemisch wurde auf 200°C aufgeheizt und dann zusätzlich mit Wasser in einer Menge von 300 ml/Std. angereichert. Dann wurde auf 350°C aufgeheizt und der Katalysator bei dieser Temperatur und 6 bar mit dem Stickstoff-Sauerstoff-Wasserdampf-Gemisch 24 Stunden behandelt. Es wurde danach auf eine Temperatur von 320°C abgekühlt und dann zusätzlich zu dem Stickstoff, Sauerstoff und Wasserdampf enthaltenden Gemisch Naphthalin gasförmig in einer Menge von 690 g/Stunde über den Katalysator gefahren. Anschließend wurde mit einer Geschwindigkeit von 6°C/Stunde die Temperatur auf 360°C erhöht. Nach einer Laufzeit des Katalysators von 500 Stunden wurden folgende Ergebnisse erhalten:

| Katalysator | Raum-Zeit-Ausbeute [g Naphthochinon pro Liter Katalysator und pro Stunde] | Raum-Zeit-Ausbeute [g Phthalsäureanhydrid pro Liter Katalysator und pro Stunde] | Gewichtsverhältnis Naphthochinon zu Phthalsäure- anhydrid |
|---|---|---|---|
| 1 | 39,1 | 50,7 | 0,77 : 1 |
| 2 | 45,8 | 46,4 | 0,98 : 1 |

Mit dem Katalysator 2 werden somit deutlich verbesserte Werte hinsichtlich der Naphthochinon-Raum-Zeit-Ausbeute und hinsichtlich des Verhältnisses von Naphthochinon zu Phthalsäureanhydrid erhalten.

### Beispiel 3

### Hydrierung von o-Nitrotoluol

### a) Vergleichskatalysator

In der DE-C-1 545 297 wird die Herstellung eines Hydrierkatalysators beschrieben. Beispiel 4 wurde derart nachgearbeitet, daß ein Katalysator mit 5 g Pd pro Liter Li-Spinell erhalten wurde. Das ergab den Katalysator 1.

### b) Erfindungsgemäßer Katalysator

Entsprechend Beispiel 24 (siehe Teil D) werden 50 ml = 65 g Träger (hergestellt entsprechend Teil D, Beispiel 15) mit 5,3 ml einer Lösung, die 1,67 g $Na_2PdCl_4$ (entsprechend 0,25 g Palladium) enthält, getränkt, mit 20 ml einer 10gew.-%igen Hydrazinlösung zur Überführung des Palladiums in den elementaren Zustand versetzt, mit demineralisiertem Wasser neutral und chloridfrei gewaschen und anschließend bei 110°C im Warmluftstrom bis zur Gewichtskonstanz getrocknet.

Es werden 50 ml eines Katalysators erhalten, der 5 g Pd pro Liter Katalysator enthält. Das ergab den Katalysator 2.

### c) Hydrierung von o-Nitrotoluol

Die Hydrierung von o-Nitrotoluol wird bei Normaldruck in einer Schüttelente mit eingebautem Körbchen zur Aufnahme des Katalysators durchgeführt. Es werden jeweils 10 ml Katalysator, 5 g o-Nitrotoluol und 35 ml Methanol bei 50°C und 0,1 bar Wasserstoffdruck eingesetzt. Gemessen wird die Zeit der Wasserstoffaufnahme.

| Katalysator | Hydrierzeit [min] | Umsatz o-Nitrotoluol [%] |
|---|---|---|
| 1 | 270 | 98,9 |
| 2 | 220 | 99,0 |

Mit Katalysator 2 werden verbesserte Werte hinsichtlich Hydrierzeit und Umsatz o-Nitrotoluol erhalten.

### Teil D

### Herstellungsbeispiele für erfindungsgemäße Produkte

### Beispiele 1—5

Gemäß Teil B, Beispiel 1, wird die Beschichtung der verschiedensten Trägermaterialien durchgeführt.

| | Beispiel Nr. | | | | | |
|---|---|---|---|---|---|---|
| | 1*) | 2 | 3 | 4 | 5 | 5a |
| Einsatzmaterialien-Nr. aus Teil A | | | | | | |
| Anorganische Grundmasse Nr. | 4 | 2 | 5 | 6 | 3 | 2 |
| Einsatzmenge [g] | 577 | 2180 | 340 | 200 | 4725 | 1070 |
| Kieselsol Nr. | 9 | 9 | 9 | 9 | 9 | 9 |
| Einsatzmenge [g] | 290 | 1667 | 444 | 288 | 1081 | 676 |
| Kieselgur Nr. | 12 | 12 | — | 12 | 12 | — |
| Einsatzmenge [g] | 87 | 250 | — | 87 | 324 | — |
| Ultrasil VN 2 Nr. | 10 | — | — | 10 | 10 | — |
| Einsatzmenge [g] | 87 | — | — | 87 | 324 | — |
| Kaliwasserglas Nr. | | | 11 | | | — |
| Einsatzmenge [g] | — | — | 319 | — | — | — |
| Vanadyloxalat-Lösung Nr. | 13 | — | — | 13 | 13 | 13 |
| Einsatzmenge [g] | 344 | — | — | 344 | 1290 | 274 |
| Kaliumsulfat [g] | 209 | — | — | 209 | 784 | 163 |
| Schwefelsäure 97% [g] | 121 | — | — | 121 | 455 | 92 |

(Fortsetzung)

| | Beispiel Nr. | | | | | |
|---|---|---|---|---|---|---|
| | 1*) | 2 | 3 | 4 | 5 | 5a |
| Wasser [g] | 146 | — | 237 | 146 | 830 | — |
| Ausbeute | | | | | | |
| [g] Produkt | 985 | — | 468 | 495 | 6505 | 1400 |
| Gewichtszunahme, bezogen auf eingesetzte anorganische Grundmasse | | | | | | |
| [g] | 71 | 480 | 128 | 295 | 1780 | 428 |
| [%] | 66 | 22 | 38 | 148 | 37,7 | 40 |
| Mittlere Schichtdicke [μm] | n. b. | 420 | n. b. | n. b. | 791**) | n. b. |

Anmerkungen
*) Die Arbeitstemperatur bei der Auftragung betrug abweichend von Teil B, Beispiel 1, 200—230°C.
**) Die Schichtdicke schwankt zwischen 2025 μm und 445 μm. Über eine Meßreihe wurde die mittlere Schichtdicke zu 791 μm bestimmt.
Solche starken Schwankungen in den Schichtdicken treten bei geometrisch regelmäßig geformten anorganischen Grundmassen, wie z. B. Kugeln, nicht auf.
n. b. bedeutet nicht bestimmt.
Die Produkte der Beispiele 1, 4, 5 und 5a sind Katalysatoren für die Naphthalin-Oxidation. Die Produkte der Beispiele 2 und 3 sind Vorstufen für die Herstellung von Katalysatoren (siehe Beispiel 24).

## Beispiele 6—12

Gemäß Teil B, Beispiel 1, wird die Herstellung von Schichten verschiedener mittlerer Dicken durchgeführt.

| Versuchsbedingungen | Beispiel Nr. | | | | | | |
|---|---|---|---|---|---|---|---|
| | 6 | 7 | 8 | 9 | 10 | 11 | 12 |
| Einsatzmaterialien-Nr. aus Teil A | | | | | | | |
| Anorganische Grundmasse Nr. | 1 | 1 | 1 | 1 | 1 | 1 | 1 |
| Einsatzmenge [g] | 1625 | 1625 | 1625 | 1600 | 1615 | 1600 | 1600 |
| Kieselsol Nr. | 9 | 9 | 9 | 9 | 9 | | |
| Einsatzmenge [g] | 41 | 112 | 722 | 1000 | 555 | — | — |
| Kieselgur Nr. | 12 | 12 | 12 | | | | |
| Einsatzmenge [g] | 12 | 34 | 217 | — | — | — | — |
| Kaliwasserglas Nr. | | | | | | 11 | 11 |
| Einsatzmenge [g] | — | — | — | — | — | 1587 | 793 |
| Ultrasil Nr. | 10 | 10 | 10 | 10 | | | |
| Einsatzmenge [g] | 12 | 34 | 217 | 100 | — | — | — |

(Fortsetzung)

| Versuchsbedingungen | Beispiel Nr. | | | | | | |
|---|---|---|---|---|---|---|---|
| | 6 | 7 | 8 | 9 | 10 | 11 | 12 |
| Aluminiumoxid Nr. | | | | | 17 | | |
| Einsatzmenge [g] | — | — | — | — | 333 | — | — |
| Zeolith Nr. | | | | | | 15 | 15 |
| Einsatzmenge [g] | — | — | — | — | — | 190 | 375 |
| Aktivkohle Nr. | | | | 14 | | | |
| Einsatzmenge [g] | — | — | — | 400 | — | — | — |
| Vanadyloxalat-Lösung Nr. | 13 | 13 | 13 | | | | |
| Einsatzmenge [g] | 49 | 133 | 862 | — | — | — | — |
| Kaliumsulfat [g] | 30 | 81 | 523 | — | — | — | — |
| Schwefelsäure 100% [g] | 17 | 47 | 304 | | | | |
| Wasser [g] | 282 | 768 | 4953 | 2500 | 1611 | 722 | 1331 |
| Ausbeute | | | | | | | |
| [g] Produkt | 1690 | 1823 | 2800 | 1651 | 1954 | 2300 | 2102 |
| Gewichtszunahme, bezogen auf eingesetzte anorganische Grundmasse | | | | | | | |
| [g] | 65 | 198 | 1175 | 51 | 339 | 700 | 502 |
| [%] | 4 | 12 | 72 | 3 | 21 | 44 | 31 |
| Mittlere Schichtdicke [µm] | 50 | 150 | 650 | 50 | 350 | n. b. | n. b. |

Anmerkungen
Die Produkte aus den Beispielen 6—8 sind für die Naphthalin-Oxidation geeignete Katalysatoren. Die Produkte aus den Beispielen 9—12 sind Katalysatorträgermaterialien. Wenn man diese entsprechend Teil C, Beispiel 3b, so mit Palladium belegt, daß sie 1,5% Palladium enthalten, erhält man Katalysatoren, die für die Hydrierung von o-Nitrotoluol nach dem in Teil C, Beispiel 3c, beschriebenen Verfahren geeignet sind.
n. b. bedeutet nicht bestimmt.

Beispiele 13—19

Gemäß Teil B, Beispiel 1, wird die Herstellung von Kieselsäureschichten durchgeführt, die unterschiedliche physikalische Eigenschaften haben.

| Versuchsbedingungen | Beispiel Nr. | | | | | | |
|---|---|---|---|---|---|---|---|
| | 13 | 14 | 15 | 16 | 17 | 18 | 19 |
| Einsatzmaterialien-Nr. aus Teil A | | | | | | | |
| Anorganische Grundmasse Nr. | 1 | 1 | 1 | 1 | 1 | 1 | 1 |
| Einsatzmenge [g] | 1615 | 1615 | 1615 | 1615 | 1615 | 1600 | 1600 |
| Kieselsol Nr. | 9 | 9 | 9 | 9 | 9 | 9 | 9 |
| Einsatzmenge [g] | 666 | 887 | 887 | 447 | 1333 | 417 | 833 |
| Kieselgur Nr. | 12 | 12 | | 12 | | | |
| Einsatzmenge [g] | 100 | 134 | — | 133 | — | — | — |
| Kaliwasserglas Nr. | | | | | | 11 | 11 |
| Einsatzmenge [g] | — | — | — | — | — | 1190 | 595 |
| Ultrasil Nr. | 10 | | 10 | 10 | | 10 | 10 |
| Einsatzmenge [g] | 100 | — | 134 | 133 | — | 125 | 125 |
| Wasser [g] | 1133 | 979 | 979 | 1287 | 667 | 768 | 947 |
| Ausbeute | | | | | | | |
| [g] Produkt | 1793 | 1906 | 1954 | 1765 | 1986 | 2192 | 1824 |
| Gewichtszunahme, bezogen auf eingesetzte anorganische Grundmasse | | | | | | | |
| [g] | 178 | 291 | 339 | 150 | 371 | 592 | 224 |
| [%] | 11,5 | 18 | 21 | 9,3 | 23 | 37 | 14 |
| Mittlere Schichtdicke [µm] | 95 | 140 | 335 | 180 | 185 | n. b. | n. b. |
| Wahre Dichte [g/cm$^3$]*) | 2,589 | 2,576 | 2,549 | 2,606 | 2,556 | 2,366 | 2,552 |
| Scheinbare Dichte [g/cm$^3$]*) | 2,210 | 2,297 | 2,027 | 2,281 | 2,273 | 1,748 | 2,389 |
| Gesamtporenvolumen [mm$^3$/g]*) | 66 | 47 | 101 | 54 | 49 | 149 | 27 |
| BET-Oberfläche [m$^2$/g]*) | 11,6 | 12,8 | 24,9 | 9,2 | 22,4 | ≪0,1 | <0,1 |
| Mittlerer Porendurchmesser [Å]*) | 230 | 150 | 160 | 230 | 90 | n. b. | n. b. |
| Porosität [%] | 14,6 | 10,8 | 20,5 | 12,5 | 11,1 | 26,1 | 6,4 |

Anmerkungen
*) Diese Werte beziehen sich auf das gesamte Produkt, d.h. anorganische Grundmasse plus Kieselsäureschicht.
Die Produkte aus den Beispielen 13—19 sind Katalysatorträgermaterialien. Wenn man diese entsprechend Teil C, Beispiel 3b, so mit Palladium belegt, daß sie 1,5% Palladium enthalten, erhält man Katalysatoren, die für die Hydrierung von o-Nitrotoluol nach dem in Teil C, Beispiel 3c, beschriebenen Verfahren geeignet sind.
n. b. bedeutet nicht bestimmt.

25

## 0 083 791

### Beispiele 20 und 20a

Durchführung gemäß Teil B, Beispiel 1; Herstellung von Katalysatoren
unter Zusatz von Porosierungsmitteln

| Versuchsbedingungen | Beispiel Nr. | |
|---|---|---|
| | 20 | 20a |
| Einsatzmaterialien-Nr. aus Teil A | | |
| Anorganische Grundmasse Nr. | 1 | 1 |
| Einsatzmenge [g] | 1615 | 16 150 |
| Kieselsol Nr. | 9 | 9 |
| Einsatzmenge [g] | 288 | 3 335 |
| Kieselgur Nr. | 12 | — |
| Einsatzmenge [g] | 86 | — |
| Kaliwasserglas Nr. | | |
| Einsatzmenge [g] | — | — |
| Ultrasil Nr. | 10 | |
| Einsatzmenge [g] | 86 | — |
| Vanadyloxalat-Lösung Nr. | 13 | 13 |
| Einsatzmenge [g] | 344 | 4 498 |
| Kaliumsulfat [g] | 209 | 2 668 |
| Schwefelsäure 97% [g] | 121 | 1 511 |
| Wasser [g] | 816 | 3 200 |
| Oxalsäuredihydrat [g] | 125 | — |
| Graphit [g] | — | 2 400 |
| Ausbeute | | |
| [g] Produkt | 2095 | 23 305 |
| Gewichtszunahme, bezogen auf eingesetzte anorganische Grundmasse | | |
| [g] | 480 | 7 155 |
| [%] | 30 | 44 |

Die so erhaltenen Produkte sind für die Oxidation von Naphthalin geeignete Katalysatoren. Sie wurden aktiviert durch Überleiten von 1 m$^3$ Luft pro Liter Katalysator während 8 Stunden bei 400° C.

Bei dem in Beispiel 20a eingesetzten Graphit handelt es sich um sogenannten Feinpudergraphit, der von der Fa. Brockhues, Niederwalluf (Bundesrepublik Deutschland), bezogen wurde. Gemäß Spezifikation hat dieser Graphit eine Teilchengröße von 95% unter 0,1 mm, einen Kohlenstoffgehalt von 85 Gew.-% und einen Aschegehalt von 15 Gew.-%.

26

Beispiele 21—23

Entsprechend Teil B, Beispiel 2, werden Produkte durch zweifache Beschichtung hergestellt.

| Versuchsbedingungen | Beispiel Nr. | | | |
|---|---|---|---|---|
| | 21 | 22 | 23a | 23b*) |
| Einsatzmaterialien-Nr. aus Teil A | | | | |
| Anorganische Grundmasse Nr. | 1 | Endprodukt aus Beispiel 21 | 8 | Endprodukt aus 23a |
| Einsatzmenge [g] | 4585 | 3290 | 820 | 730 |
| Kieselsol Nr. | 9 | 9 | — | 9 |
| Einsatzmenge [g] | 1000 | 1081 | — | 333 |
| Kieselgur Nr. | | 12 | — | |
| Einsatzmenge [g] | — | 324 | | |
| Kaliwasserglas Nr. | 11 | | 11 | 11 |
| Einsatzmenge [g] | 2857 | — | 1250 | 952 |
| Ultrasil Nr. | 10 | 10 | | |
| Einsatzmenge [g] | 300 | 324 | — | |
| Ionenaustauscher Nr. | | | | 16 |
| Einsatzmenge [g] | | | | 100 |
| Vanadyloxalat-Lösung Nr. | | 13 | | |
| Einsatzmenge [g] | — | 1290 | — | |
| Kaliumsulfat [g] | — | 784 | — | |
| Schwefelsäure 100% [g] | — | 455 | — | |
| Wasser [g] | 1843 | 829 | 500 | 524 |
| Ausbeute | | | | |
| [g] Produkt | 6025 | 4743 | 1060 | 1260 |
| Gewichtszunahme, bezogen auf eingesetzte anorganische Grundmasse | | | | |
| [g] | 1440 | 1453 | 240 | 530 |
| [%] | 31 | 44 | 29 | 73 |

Anmerkungen
Das Produkt aus Beispiel 22 ist ein Katalysator, der für die Oxidation von Naphthalin geeignet ist. Das Produkt aus Beispiel 23b ist ein Katalysatorträgermaterial. Wenn man dieses mit 1,5% Palladium belegt (siehe Teil C, Beispiel 3b), so erhält man einen für die Hydrierung von o-Nitrotoluol geeigneten Katalysator.
*)    Die Beschichtung erfolgte bei 130—150°C, bedingt durch Instabilität des Ionenaustauschers bei höheren Temperaturen.

Beispiele 24 bis 29

Verschiedene erfindungsgemäße Produkte, die mit einer Kieselsäure enthaltenden Schicht überzogen sind, werden mit verschiedenen Wirkstoffen beladen.

Herstellung und Eigenschaften der Ausgangsprodukte

|  | Herstellung | Saugfähigkeit [g Wasser/100 g Ausgangsmaterial] | Schüttgewicht [g/l Ausgangsmaterial] |
|---|---|---|---|
| Für Beispiel 24 | gemäß Teil D, Beispiel 15 | 8,2 | 1296 |
| Für Beispiele 25 bis 29 | gemäß Teil B, Beispiel 1 | 4,2 | 1428 |

## Beispiel 24

50 ml = 65 g des o. a. Ausgangsproduktes werden mit 5,3 ml einer Lösung, die 6,9 g $Na_2PdCl_4$-Lösung (entsprechend 1 g Palladium) enthält, getränkt, mit 20 ml einer 10gew.-%igen Hydrazinlösung zur Überführung des Palladiums in den elementaren Zustand versetzt, mit demineralisiertem Wasser neutral und chloridfrei gewaschen und anschließend bei 110° C im Warmluftstrom bis zur Gewichtskonstanz getrocknet.

Es werden 50 ml eines Katalysators erhalten, der 20 g Pd pro Liter Katalysator enthält. Dieser Katalysator eignet sich für die Hydrierung von o-Nitrotoluol.

## Beispiel 25

100 ml = 143 g des o. a. Ausgangsproduktes werden mit 6,0 ml einer Lösung, die 1,645 g Rutheniumnitrat-Hydrat (entsprechend 0,5 g Ru) enthält, getränkt, getrocknet und bei 400° C getempert. Das so abgeschiedene Rutheniumoxid wird durch Behandlung mit Wasserstoff bei erhöhter Temperatur zum Metall reduziert.

Es werden 100 ml des Katalysators erhalten, der 5 g Ru pro Liter Katalysator enthält. Dieser Katalysator eignet sich für die Hydrierung von o-Nitrotoluol.

## Beispiel 26

100 ml = 143 g des o. a. Ausgangsproduktes werden mit 6,0 ml einer Lösung, die 7,5 g $NiCl_2 \cdot 6 H_2O$ (entsprechend 1,85 g Ni) enthält, getränkt und bei 80° C im Vakuum bis zur Gewichtskonstanz getrocknet.

Es werden 100 ml eines Katalysators erhalten, der 18,5 g Ni in Form von Nickelchlorid pro Liter Katalysator enthält.

## Beispiel 27

100 ml = 143 g des o. a. Ausgangsproduktes werden mit 6,0 ml einer Lösung, die 10,0 g $Cu(NO_3)_2 \cdot 3 H_2O$ (entsprechend 2,6 g Cu) enthält, getränkt und bei 80° C im Vakuum bis zur Gewichtskonstanz getrocknet.

Es werden 100 ml eines Katalysators erhalten, der 26 g Cu in Form von Kupfernitrat pro Liter Katalysator enthält.

## Beispiel 28

100 ml = 143 g des o. a. Ausgangsproduktes werden mit 6,0 ml einer Lösung, die 10,0 g $Co(NO_3)_2 \cdot 6 H_2O$ (entsprechend 2 g Co) enthält, getränkt und bei 80° C im Vakuum bis zur Gewichtskonstanz getrocknet.

Es werden 100 ml eines Katalysators erhalten, der 20 g Co in Form von Kobaltnitrat pro Liter Katalysator enthält.

## Beispiel 29

100 ml = 143 g des o. a. Ausgangsproduktes werden mit 6,0 ml einer Lösung, die 6,0 g $FeCl_3 \cdot 6 H_2O$ (entsprechend 1,24 g Fe) enthält, getränkt und bei 80°C im Vakuum bis zur Gewichtskonstanz getrocknet.

Es werden 100 ml eines Katalysators erhalten, der 1,24 g Fe in Form von Eisenchlorid pro Liter Katalysator enthält.

Bei den Produkten aus den Beispielen 26—29 handelt es sich um Katalysatoren, die noch durch Wasserstoffbehandlung zu aktivieren sind. Die so aktivierten Katalysatoren können für die Hydrierung von o-Nitrotoluol eingesetzt werden.

## Teil E

### Beschreibung von Herstellungsmethoden für erfindungsgemäße Produkte unter Verwendung organischer Grundmassen

## Beispiel 30

In einer 50 l fassenden, heizbaren Dragiertrommel werden 341 g Polystyrol (Material Nr. 18, s. Teil A) vorgelegt und bei einer Umdrehungsgeschwindigkeit von 4 U/min auf 70°C aufgeheizt. Währenddessen werden in einem 3-l-Behälter 2500 g Kieselsol und 250 g Kaliwasserglas (Materialien Nr. 9 und 11, s. Teil A) unter Rühren zusammengegeben. Diese Mischung wird unter stetigem Rühren pneumatisch aus einem Druckbehälter heraus auf das ständig bewegte, zu Beginn der Auftragung 70°C heiße, vorgelegte Polystyrol aufgesprüht. Sobald die Temperatur des bewegten Materials 60°C unterschritten hat, wird der Sprühprozeß unterbrochen, bis 70°C wieder erreicht sind. Dieser Vorgang wird viermal durchgeführt. Dann erfolgen vier Auftragungen im Temperaturbereich von 80 bis 70°C, sechs im Temperaturbereich 100 bis 85°C, sechs im Temperaturbereich 120 bis 100°C und die restlichen im Temperaturbereich 150 bis 100°C.

Man läßt nach beendeter Auftragung das Produkt bei einer Umdrehungsgeschwindigkeit von 1 U/min auf 40°C abkühlen und entleert die Trommel.

Es werden 763 g Produkt, enthaltend 422 g Kieselsäure in einer Schale, erhalten. Die Saugfähigkeit des Produktes beträgt 12,48% bei einem Schüttgewicht von 785 g/l und einer Restfeuchte <1%.

Das so erhaltene Produkt wird zur Entfernung des Polystyrols in einem Muffelofen getempert, wobei die Temperatur mit 50°C/h auf 700°C gesteigert wird. Bei dieser Temperatur wird das Einsatzprodukt so lange gehalten, bis der gesamte organische Anteil ausgetrieben ist. Das erkennt man daran, daß Gewichtskonstanz erreicht wird.

Es werden 417 g Produkt erhalten entsprechend 98,8% der Theorie.

## Beispiele 31 bis 37

Gemäß Teil E, Beispiel 30, wird die Beschichtung der verschiedensten Trägermaterialien durchgeführt.

| | Beispiel Nr. | | | | | | |
| | 31 | 32 | 33 | 34 | 35 | 36 | 37 |
|---|---|---|---|---|---|---|---|
| Organische Grundmasse (Nr. s. Teil A) | 19 | 20 | 21 | 22 | 23 | 24 | 25 |
| Einsatzmenge [g] | 463 | 608 | 390 | 730 | 386 | 778 | 864 |
| Kieselsol (Nr. s. Teil A) | 9 | 9 | 9 | 9 | 9 | 9 | 9 |
| Einsatzmenge [g] | 600 | 417 | 500 | 833 | 500 | 1399 | 1555 |
| Kieselsäure (Nr. s. Teil A) | 10 | 10 | 10 | 10 | 10 | 10 | 10 |
| Einsatzmenge [g] | 180 | 208 | 155 | 250 | 150 | 420 | 467 |
| Wasserglas (Nr. s. Teil A) | 11 | 11 | 11 | 11 | 11 | 11 | 11 |
| Einsatzmenge [g] | 1714 | 1984 | 1400 | 2381 | 1428 | 2000 | 2222 |

(Fortsetzung)

| | Beispiel Nr. | | | | | | |
|---|---|---|---|---|---|---|---|
| | 31 | 32 | 33 | 34 | 35 | 36 | 37 |
| Wasser [g] | 360 | 250 | 300 | 1536 | 300 | 900 | 1000 |
| Ausbeute [g Produkt] | 1111 | 1338 | 780 | 1644 | 938 | 1634 | 1970 |
| Gewichtszunahme, bezogen auf eingesetzte org. Grundmasse | | | | | | | |
| [g] | 648 | 730 | 390 | 914 | 551 | 856 | 1106 |
| [%] | 140 | 120 | 100 | 125 | 142 | 110 | 128 |
| Temperung des beschichteten org. Trägers | | | | | | | |
| max. Temp. [°C] | 700 | 700 | 700 | 700 | 700 | 700 | 700 |
| Gewichtsabnahme [%] | 35 | 40 | 45 | 24 | 43 | 50 | 30 |

## Beispiel 38

Entsprechend Teil B, Beispiel 1, werden 393 g Material, erhalten gemäß Beispiel 30, jedoch ohne Entfernung des Polystyrols, mit 1489 g einer aus 337 g Vanadyloxalat-Lösung (Material Nr. 13, s. Teil A), 113 g 98%ige Schwefelsäure, 201 g Kaliumsulfat und 8337 g Kieselsol (Material Nr. 9, s. Teil A) innerhalb von 4 Stunden besprüht. Es werden 725 g Produkt erhalten, was einer Gewichtszunahme, bezogen auf das Einsatzmaterial, von 85% oder 332 g entspricht. Das so erhaltene Produkt wurde 17 h bei 400°C getempert, wobei diese Temperatur innerhalb von 8 h erreicht wurde.

Hiernach wurden 507 g, entsprechend 30% Gewichtsabnahme, als Endprodukt erhalten, welches als Katalysator für die Oxidation von Naphthalin Verwendung finden kann.

## Patentansprüche

1. Kieselsäurehaltige Formkörper, dadurch gekennzeichnet, daß ein Hohlraum oder eine stückige anorganische Grundmasse von einer poröse Kieselsäure enthaltenden Schicht umgeben ist.

2. Kieselsäurehaltige Formkörper nach Anspruch 1, dadurch gekennzeichnet, daß die Kieselsäure enthaltende Schicht katalytisch aktive Substanzen oder Vorläufer davon enthält.

3. Verfahren zur Herstellung von kieselsäurehaltigen Formkörpern, dadurch gekennzeichnet, daß man Kieselsol oder ein Gemisch, enthaltend Wasser, Kieselsol und/oder Wasserglas und gegebenenfalls feinpulvrige, wasserunlösliche Kieselsäure und/oder Porosierungsmittel auf eine stückige Grundmasse aufbringt, wobei die Temperatur der Grundmasse zu Beginn des Aufbringens des Kieselsols oder des Gemisches unter dem Erweichungspunkt der Grundmasse und im weiteren Verlauf des Aufbringens des Kieselsols oder des Gemisches über der Siedetemperatur des Wassers liegt, das Kieselsol oder das Gemisch so zugegeben wird, daß das Wasser schnell verdampft und der Wassergehalt der Grundmasse stets unter 5 Gew.-% beträgt und, im Falle des Einsatzes von organischen Grundmassen, diese anschließend durch Erhitzen auf Temperaturen im Bereich von 200 bis 1300°C entfernt.

4. Verfahren nach Anspruch 3, dadurch gekennzeichnet, daß man eine anorganische Grundmasse, die zumindest an der Oberfläche eine Saugfähigkeit von unter 10 g Wasser pro 100 g und eine BET-Oberfläche von unter 5 m²/g aufweist, und eine feinpulvrige, wasserunlösliche Kieselsäure, die eine Primär-Teilchengröße im Bereich von 1 bis 200 nm und eine Agglomeratgröße im Bereich von 0,5 bis 100 μm aufweist, und/oder Kieselgur einsetzt und das Kieselsol oder das Gemisch bei Temperaturen oberhalb des Siedepunktes von Wasser aufbringt.

5. Verfahren nach Anspruch 4, dadurch gekennzeichnet, daß während des Aufbringens des Kieselsols oder des Gemisches die Temperatur der anorganischen Grundmasse im Bereich 105 bis 800°C liegt.

6. Verfahren nach Anspruch 3, dadurch gekennzeichnet, daß man eine organische Grundmasse einsetzt, die Temperatur der organischen Grundmasse zu Beginn des Aufbringens des Kieselsols oder des Gemisches unter dem Erweichungspunkt hält, das weitere Aufbringen von Kieselsol oder Gemisch

30

bei Temperaturen im Bereich 105 bis 220°C durchführt und anschließend die organische Grundmasse durch Erhitzen auf Temperaturen im Bereich von 200 bis 1300°C entfernt.

7. Verfahren nach Ansprüchen 3 bis 6, dadurch gekennzeichnet, daß man Kieselsol oder ein Gemisch aufbringt, bis sich eine kieselsäurehaltige Schicht einer Dicke im Bereich von 10 bis 3000 µm gebildet hat.

8. Verfahren nach Ansprüchen 3 bis 7, dadurch gekennzeichnet, daß man katalytisch aktive Substanzen oder Vorläufer davon zusammen mit Kieselsol oder dem Kieselsäure enthaltenden Gemisch aufbringt oder zunächst ein Gemisch aufbringt, das eine von katalytisch aktiven Substanzen oder Vorläufern davon freie kieselsäurehaltige Schicht ergibt und anschließend Lösungen oder Suspensionen von katalytisch aktiven Substanzen oder Vorläufern davon in die Schicht einbringt.

9. Verwendung von kieselsäurehaltigen Formkörpern, bei denen ein Hohlraum oder eine stückige anorganische Grundmasse von einer poröse Kieselsäure enthaltenden Schicht umgeben ist, als Trägermaterial für Katalysatoren.

10. Verwendung von kieselsäurehaltigen Formkörpern, bei denen ein Hohlraum oder eine stückige anorganische Grundmasse von einer poröse Kieselsäure enthaltenden Schicht umgeben ist, die katalytisch aktive Substanzen enthält, als Katalysatoren.

## Claims

1. Silica-containing shaped articles, characterised in that a hollow space or a lumpy inorganic base material is surrounded by a layer containing porous silica.

2. Silica-containing shaped articles according to Claim 1, characterised in that the silica-containing layer contains catalytically active substances or precursors thereof.

3. Process for preparing silica-containing shaped articles, characterised in that silica sol or a mixture containing water, silica sol and/or waterglass and, if appropriate, finely pulverulent, water-insoluble silica and/or porosity-forming agents is applied to a lumpy base material while the temperature of the base material is, at the start of the application of the silica sol or of the mixture, below the softening point of the base material and, as the application of the silica sol or of the mixture proceeds, above the boiling temperature of water, and the silica sol or the mixture is added in such a way that the water evaporates rapidly and the water content of the base material is always less than 5% by weight, and, if use is made of organic base materials, these organic base materials are then removed by heating to temperatures within the range of 200 to 1,300° C.

4. Process according to Claim 3, characterised in that an inorganic base material which, at least at the surface, has an absorbency of less than 10 g of water per 100 g and a BET surface area of less than 5 m²/g and a finely pulverulent water-insoluble silica shich has a primary particle size in a range of 1 to 200 nm and an agglomerate size within a range of 0.5 to 100 µm and/or kieselguhr are used, and the silica sol or the mixture is applied at temperatures above the boiling point of water.

5. Process according to Claim 4, characterised in that the temperature of the inorganic base material is within a range of 105 to 800° C during the application of the silica sol or of the mixture.

6. Process according to Claim 3, characterised in that an organic base material is used, the temperature of the organic base material at the start of the application of the silica sol or of the mixture is kept below its softening point, as the application of silica sol or mixture proceeds the process is carried out at temperatures within a range of 105 to 220°C, and the organic base material is then removed by heating to temperatures within the range of 200 to 1,300° C.

7. Process according to Claims 3 to 6, characterised in that silica sol or a mixture is applied until a silica-containing layer having a thickness within a range of 10 to 3000 µm has formed.

8. Process according to Claims 3 to 7, characterised in that catalytically active substances or precursors thereof are applied together with silica sol or with the mixture containing silica or a mixture is first applied which produces a silica-containing layer free of catalytically active substances or precursors thereof and solutions or suspensions of catalytically active substances or precursors thereof are then introduced into the layer.

9. Use of silica-containing shaped articles in which a hollow space or a lumpy inorganic base material is surrounded by a layer containing porous silica, as support material for catalysts.

10. Use of silica-containing shaped articles in which a hollow space or a lumpy inorganic base material is surrounded by a layer containing porous silica and catalytically active substances, as catalysts.

## Revendications

1. Pièces moulées contenant de la silice, caractérisées en ce qu'une cavité ou une matière de base inorganique en morceaux est entourée d'une couche contenant de la silice poreuse.

2. Pièces moulées contenant de la silice suivant la revendication 1, caractérisées en ce que la couche contenant de la silice renferme des substances catalytiquement actives ou leurs précurseurs.

3. Procédé de production de pièces moulées contenant de la silice, caractérisé en ce qu'on applique un sol de silice ou un mélange contenant de l'eau, und sol de silice et/ou du verre soluble et, le cas échéant, de la silice en poudre fine insoluble dans l'eau et/ou un agent de porosité sur une matière de base en morceaux, la température de la matière de base étant inférieure au point de ramollissement de cette matière au début de l'application du sol de silice ou du mélange et étant au-dessus de la température d'ébullition de l'eua pendant la poursuite de l'application du sol de silice ou du mélange, le sol de silice ou le mélange est ajouté de telle manière que l'eau s'évapore rapidement et que la teneur en eau de la matière de base s'élève constamment à une valeur inférieure à 5% en poids et, dans le cas de l'utilisation de matières organiques de base, les matières sont ensuite éliminées par chauffage à des températures comprises dans l'intervalle de 200 à 1300° C.

4. Procédé suivant la revendication 3, caractérisé en ce qu'on utilise une matière inorganique de base qui présente au moins à la surface un pouvoir absorbant inférieur à 10 g d'eau par 100 g et une surface BET de moins de 5 m²/g et une silice en poudre fine insoluble dans l'eau qui présente un diamètre primaire des particules compris dans l'intervalle de 1 à 200 nm et un diamètre des particules agglomérées dans l'intervalle de 0,5 à 100 μm et/ou du kieselguhr et le sol de silice ou le mélange est appliqué à des températures supérieures au point d'ébullition de l'eau.

5. Procédé suivant la revendication 4, caractérisé en ce que pendant l'application du sol de silice ou du mélange, la température de la matière inorganique de base se situe dans l'intervalle de 105 à 800° C.

6. Procédé suivant la revendication 3, caractérisé en ce qu'on utilise une matière organique de base, on maintient la température de la matière organique de base au début de l'application du sol de silice ou du mélange au-dessous du point de ramollissement, on poursuit l'application du sol de silice ou du mélange à des températures comprises dans l'intervalle de 105 à 220° C puis on enlève la matière organique de base par chauffage à des températures comprises dans l'intervalle de 200 à 1300° C.

7. Procédé suivant les revendications 3 à 6, caractérisé en ce qu'on applique un sol de silice ou un mélange jusqu'à ce qu'une couche contenant de la silice, d'une épaisseur de 10 à 3000 μm, se soit formée.

8. Procédé suivant les revendications 3 à 7, caractérisé en ce qu'on applique des substances douées d'activité catalytique ou leurs précurseurs conjointement avec un sol de silice ou le mélange contenant de la silice ou bien on applique tout d'abord un mélange qui donne une couche contenant de la silice dépourvue de substances douées d'activité catalytique ou de leurs précurseurs, puis on introduit dans la couche des solutions ou suspensions de substances ou précurseurs de substances douées d'activité catalytique.

9. Utilisation de pièces moulées contenant de la silice, dans lesquelles une cavité ou une matière de base inorganique en morceaux est entourée d'une couche poreuse contenant de la silice, comme matière de support pour catalyseurs.

10. Utilisation de pièces moulées contenant de la silice dans lesquelles une cavité ou une matière de base inorganique en morceaux est entourée d'une couche contenant de la silice poreuse, qui renferme des substances douées d'activité catalytique, comme catalyseurs.